(19) Europäisches Patentamt
European Patent Office
Office européen des brevets

(11) Veröffentlichungsnummer : 0 274 346 B1

# (12) EUROPÄISCHE PATENTSCHRIFT

(45) Veröffentlichungstag der Patentschrift : **06.03.91 Patentblatt 91/10**

(51) Int. Cl.$^5$ : **C07F 9/547,** A61K 31/675

(21) Anmeldenummer : **87810665.7**

(22) Anmeldetag : **16.11.87**

## (54) Neue Substituiertaminomethandiphosphonsäuren.

(30) Priorität : **21.11.86 CH 4665/86**

(43) Veröffentlichungstag der Anmeldung : **13.07.88 Patentblatt 88/28**

(45) Bekanntmachung des Hinweises auf die Patenterteilung : **06.03.91 Patentblatt 91/10**

(84) Benannte Vertragsstaaten : **AT BE CH DE ES FR GB GR IT LI LU NL SE**

(56) Entgegenhaltungen :
**EP-A- 0 186 405**
**JP-A-54 135 724**

(73) Patentinhaber : **CIBA-GEIGY AG**
**Klybeckstrasse 141**
**CH-4002 Basel (CH)**

(72) Erfinder : **Widler, Leo, Dr.**
**Melchior-Berristrasse 11**
**D-4142 Münchenstein (CH)**
Erfinder : **Jaeggi, Knut A., Dr.**
**General Guisan-Strasse 44**
**D-4054 Basel (CH)**

**EP 0 274 346 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Substituiertaminomethandiphosphonsäuren, insbesondere Heteroarylaminomethandiphosphonsäuren der Formel

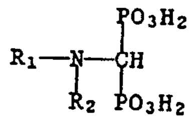

(I)

worin $R_1$ einen C-unsubstituierten oder durch Niederalkyl, durch Niederalkoxy, durch unsubstituiertes oder durch Niederalkyl, Niederalkoxy und/oder Halogen mono- oder disubstituiertes Phenyl, durch Hydroxy, durch Diniederalkylamino, durch Niederalkylthio und/oder durch Halogen C-mono- oder C-disubstituierten und/oder an einem gegebenenfalls vorhandenen substituierbaren N-Atom unsubstituiertes oder vorzugsweise durch Niederalkyl oder durch unsubstituiertes oder durch Niederalkyl, Niederalkoxy und/oder Halogen mono- oder disubstituiertes Phenylniederalkyl N-monosubstituierten Imidazolyl-, Benzimidazolyl-, 2H-1,2,3- oder 4H-1,2,4-Triazolyl-, Tetrazolyl-, Oxazolyl-, Benzoxazolyl-, Oxadiazolyl-, Thiazolyl-, Benzthiazolyl-oder Thiadiazolylrest bedeutet und $R_2$ für Wasserstoff oder Niederalkyl steht, und ihre Salze, Verfahren zur Herstellung der erfindungsgemässen Verbindung, diese oder Verbindungen der Formel I, worin $R_1$ einen unsubstituierten oder durch Niederalkyl und/oder Halogen mono- oder disubstituierten Pyrazol-3-ylrest oder Isoxazol-3-ylrest bedeutet und $R_2$ Wasserstoff darstellt, oder jeweils ein pharmazeutisch verwendbares Salz davon enthaltende pharmazeutische Präparate und ihre Verwendung als Arzneimittel-wirkstoffe. Imidazolyl ist z.B. Imidazol-2-yl oder 4-yl ; Thiazolyl ist z.B. Thiazol-2-yl, ferner Thiazol-5-yl oder -4-yl ; Oxazolyl ist z.B. Oxazol-2-yl, ferner Oxazol-4-yl ; Triazolyl ist z.B. 4H-1,2,4Triazol-3-yl oder 2H-1,2,3-Triazol-4-yl ; Tetrazolyl ist z.B. Tetrazol-5-yl ; Thiadiazolyl ist z.B. 1,2,5-Thiadiazol-3-yl ; Oxadiazolyl ist z.B. 1,3,4-Oxadiazol-2-yl ; Benzimidazolyl ist z.B. Benzimidazol-2-yl ; Benzoxazolyl ist z.B. Benzoxazol-2-yl ; Benzthiazolyl ist z.B. Benzthiazol-2-yl. Die genannten Reste können zwei gleiche oder verschiedene der eingangs genannten Substituenten aufweisen. Reste $R_1$ mit substituierbaren N-Atomen sind vorzugsweise wie angegeben N-substituiert. Reste $R_1$ sind beispielsweise 1-$C_1$-$C_4$-Alkylimidazol-2-ylreste, wie 1-Methylimidazol-2-yl, 1-Phenyl-$C_1$-$C_4$-alkylimidazol-2-ylreste, wie 1-Benzylimidazol-2-yl, Oxazol-2-yl, Thiazol-2-yl, 4-und 5-$C_1$-$C_4$-Alkylthiazol-2-ylreste, wie 4- oder 5-Methylthiazol-2-yl, 5-Phenylthiazol-2-yl, 1,2,4-Thiadiazol-5-yl, 3-Phenyl-1,2,4-thiadiazol-5-yl, 1,3,4-Thiadiazol-2-yl, 5-Methyl-1,3,4-thiadiazol-2-yl, Benzoxazol-2-yl und Benzthiazol-2-yl.

Nachstehend sind unter niederen Resten und Verbindungen solche zu verstehen, die bis und mit 7, insbesondere bis und mit 4, C-Atome aufweisen. Ferner haben die Allgemeinbegriffe beispielsweise folgende Bedeutungen :

Niederalkyl ist beispielsweise $C_1$-$C_4$-Alkyl, wie Methyl, Aethyl, Propyl oder Butyl, ferner Iso-, Sekundär- oder Tertiärbutyl, kann aber auch eine $C_5$-$C_7$-Alkyl-, wie Pentyl-, Hexyl- oder Heptylgruppe sein.

Phenylniederalkyl ist beispielsweise Phenyl-, vor allem 1-Phenyl-$C_1$-$C_4$-alkyl, wie Benzyl.

Niederalkoxy ist beispielsweise $C_1$-$C_4$-Alkoxy, wie Methoxy, Aethoxy, Propyloxy, Isopropyloxy, Butyloxy, Isobutyloxy, Sekundärbutyloxy oder Tertiärbutyloxy.

Diniederalkylamino ist beispielsweise Di-$C_1$-$C_4$-Alkylamino, wie Dimethylamino, Diäthylamino, N-Aethyl-N-methyl-amino, Dipropylamino, N-Methyl-N-propyl-amino oder Dibutylamino.

Niederalkylthio ist beispielsweise $C_1$-$C_4$-Alkylthio, wie Methylthio, Aethylthio, Propylthio oder Butylthio, ferner Iso-, Sekundär- oder Tertiärbutylthio.

Halogen ist beispielsweise Halogen der Atomnummer bis und mit 35, wie Fluor, Chlor oder Brom.

Salze von Verbindungen der Formel I sind insbesondere deren Salze mit pharmazeutisch verwendbaren Basen, wie nicht-toxische, von Metallen der Gruppen Ia, Ib, IIa und IIb abgeleitete Metallsalze, z.B. Alkalimetall-, insbesondere Natrium- oder Kaliumsalze, Erdalkalimetall-, insbesondere Calcium- oder Magnesiumsalze, Kupfer- oder Zinksalze, ebenso Ammoniumsalze mit Ammoniak oder organischen Aminen oder quaternären Ammoniumbasen, wie gegebenenfalls C-hydroxylierten aliphatischen Aminen, insbesondere Mono-, Di-oder Triniederalkylaminen, z.B. Methyl-, Aethyl-, Dimethyl- oder Diäthylamin, Mono-, Di- oder Tri-(hydroxyniederalkyl)-aminen, wie Aethanol-, Diäthanoloder Triäthanolamin, Tris(hydroxymethyl)-amino-methan oder 2-Hydroxytertiärbutylamin, oder N-(Hydroxyniederalkyl)-N,N-diniederalkylaminen bzw. N-(Polyhydroxyniederalkyl)-N-niederalkylaminen, wie 2-(Dimethylamino)-äthanol oder D-Glucamin, oder quaternären aliphatischen Ammoniumhydroxiden, z.B. Tetrabutylammoniumhydroxid.

In diesem Zusammenhang ist auch zu erwähnen, dass die Verbindungen der Formel I in Form innerer Salze, beispielsweise der Formel

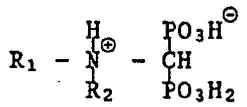 (I'),

vorliegen können. Die genannten Verbindungen können dementsprechend auch durch Behandlung mit einer starken Protonensäure, wie einer Halogenwasserstoffsäure, Schwefelsäure, Sulfonsäure, z.B. Methanoder p-Toluolsulfonsäure, oder Sulfaminsäure, z.B. N-Cyclohexylsulfaminsäure, in die entsprechenden Säureadditionssalze der Formel

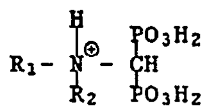 A$^{\ominus}$ (I")

überführt werden, worin A$^{\ominus}$ das Anion der Protonensäure darstellt.

Die Verbindungen der Formel I und ihre Salze weisen wertvolle pharmakologische Eigenschaften auf. Insbesondere besitzen sie eine ausgeprägte regulierende Wirkung auf den Calcium-Stoffwechsel von Warmblütern. Insbesondere bewirken sie an der Ratte eine ausgeprägte Hemmung der Knochenresorption, die sich sowohl in der Versuchsanordnung gemäss Acta Endocrinol. 78, 613 (1975) anhand des PTH-induzierten Anstieges des Serumcalciumspiegels nach subcutaner Applikation in Dosen von etwa 0,01 bis etwa 1,0 mg/kg, als auch im TPTX (Thyroparathyroidectomised) - Rattenmodell anhand der durch Vitamin D3 ausgelösten experimentellen Hypercalcämie nach Gabe von Dosen von etwa 0,001 bis 1.0 mg s.c. zeigen lässt. Ebenso wird die durch Walker-256-Tumore induzierte Tumorhyperkalzämie nach peroraler Verabreichung von etwa 1,0 bis etwa 100 mg/kg gehemmt. Ferner zeigen sie in der Adjuvansarthritis der Ratte in der Versuchsanordnung nach Newbould, Brit. J. Pharmacology 21, 127 (1963) sowie nach Kaibara et al., J. Exp. Med. 159, 1388-96 (1984) in Dosen von etwa 0,01 bis 1,0 mg/kg s.c. eine deutliche Hemmung des Fortschreitens chronisch-arthritischer Prozesse. Die Verbindungen der Formel I und ihre Salze sind deshalb vorzüglich geeignet als Arzneimittelwirkstoffe für die Behandlung von Erkrankungen, die mit Störungen des Calciumstoffwechsels in Verbindung gebracht werden können, beispielsweise entzündlicher Prozesse in Gelenken, degenerativer Prozesse im Gelenkknorpel, von Osteoporosis, Periodontitis, Hyperparathyreoidismus und von Calciumablagerungen in Blutgefässen oder an prothetischen Implantaten. Günstig beeinflusst werden sowohl Erkrankungen, bei denen eine anomale Ablagerung schwerlöslicher Calciumsalze festzustellen ist, wie solche aus den Formenkreisen der Arthritis, z.B. Morbus Bechterew, der Neuritis, der Bursitis, Periodontitis und der Tendinitis, der Fibrodysplasie, der Osteoarthrose oder der Artereosklerose, als auch solche, bei denen eine anomale Auflösung harter Körpergewebe im Vordergrund steht, wie die hereditäre Hypophosphatasie, degenerative Prozesse im Gelenkknorpel, Osteoporosen verschiedener Genese, morbus Paget und die Osteodystrophia fibrosa, ebenso durch Tumore ausgelöste osteolytische Prozesse.

Aus der japanischen Patentanmeldung Nr. A-54 135 724 war bereits ein Herstellungsverfahren u.a. für 1-(Pyrimidin-2-ylamino)methan-und 1-(3-Methylpyrazol-4-in-5-ylamino)methan-1,1-diphosphonsäure bekannt. Ferner lehrt die europäische Patentanmeldung A-186 405 dass Pyridylamino-, Pyridazinylamino-, Pyrimidinylamino- und Pyrazinaminoalkan-1,1-diphosphonsäuren eine regulierende Wirkung auf den Calcium-Stoffwechsel aufweisen und sich dementsprechend für die Behandlung von auf Störungen desselben zurückzuführenden Erkrankungen eignen. Diesen gegenüber weisen die erfindungsgemäss bereitgestellten 1-Azolylaminomethan-1,1-diphosphonsäuren den überraschenden Vorteil einer wesentlich stärkeren Wirkung auf. So wurde in dem erwähnten TPTX-Rattenmodell bei subkutaner Applikation beispielsweise für die erfindungsgemäss bereitgestellte 1-(1-Methylimidazol-2-ylamino)methan-1,1-diphosphonsäure ein $ED_{50}$-Wert von 0,00065 mg/kg und für die 1-(Pyrimidin-2-ylamino)methan-1,1-diphosphonsäure ein $ED_{50}$-Wert von 0,01 mg/kg ermittelt.

Die Erfindung betrifft insbesondere Verbindungen der Formel I, worin $R_1$ einen unsubstituierten oder durch $C_1$-$C_4$-Alkyl, wie Methyl, oder durch einen unsubstituierten oder durch $C_1$-$C_4$-Alkyl, wie Methyl, $C_1$-$C_4$-Alkoxy, wie Methoxy, und/oder Halogen, wie Chlor, mono- oder disubstituierten Phenylrest C-substituierten Thiazolyl-, wie Thiazol-2-ylrest, Benzthiazol-2-ylrest, Thiadiazolyl-, wie 1,2,4-Thiadiazol-5-yl- oder 1,3,4-Thiadiazol-2-ylrest, Oxazolyl-, wie Oxazol-2-ylrest, oder Benzoxazol-2-ylrest oder durch einen $C_1$-$C_4$-Alkyl, wie Methyl, oder durch einen unsubstituierten oder durch $C_1$-$C_4$-Alkyl, wie Methyl, $C_1$-$C_4$-Alkoxy, wie Methoxy, und/oder Halogen, wie Chlor, mono-oder disubstituierten Phenyl-$C_1$-$C_4$-alkyl-, wie Benzylrest N-substituierten Imidazol-, wie Imidazol-2-ylrest oder Imidazol-4-ylrest, oder Benzimidazol-2-ylrest, bedeutet

und $R_2$ für Wasserstoff steht, und ihre Salze, insbesondere ihre inneren Salze und pharmazeutisch verwendbaren Salze mit Basen.

Die Erfindung betrifft vor allem Verbindungen der Formel I, worin $R_1$ einen unsubstituierten oder durch $C_1$-$C_4$-Alkyl, wie Methyl, $C_1$-$C_4$-Alkoxy, wie Methoxy, Phenyl, Hydroxy, Di-$C_1$-$C_4$-Alkylamino, wie Dimethylamino oder Diäthylamino, $C_1$-$C_4$-Alkylthio, wie Methylthio, oder Halogen der Atomnummer bis und mit 35, wie Chlor, substituierten Thiazolyl-, wie Thiazol-2-yl- oder -4-ylrest, bedeutet und $R_2$ für Wasserstoff steht, und ihre Salze, insbesondere ihre inneren Salze und pharmazeutisch verwendbaren Salze und Basen.

Die Erfindung betrifft insbesondere Verbindungen der Formel I, worin $R_1$ einen unsubstituierten oder durch $C_1$-$C_4$-Alkyl, wie Methyl, durch $C_1$-$C_4$-Alkoxy, wie Methoxy, durch Phenyl, durch Hydroxy, durch Di-$C_1$-$C_4$-Alkylamino, wie Dimethylamino oder Diäthylamino, durch $C_1$-$C_4$-Alkylthio, wie Methylthio, oder durch Halogen der Atomnummer bis und mit 35, wie Chlor, C-substituierten Thiazolyl-, wie Thiazol-2-ylrest oder einen 1-$C_1$-$C_4$-Alkyl-, wie Methylimidazol-2-yl- oder -4-ylrest bedeutet und $R_2$ für Wasserstoff steht, und ihre Salze, insbesondere ihre inneren Salze und pharmazeutisch verwendbaren Salze mit Basen.

Die Erfindung betrifft in allererster Linie Verbindungen der Formel I, worin $R_1$ einen unsubstituierten oder, insbesondere in 4-und/oder 5-Stellung, durch $C_1$-$C_4$-Alkyl, wie Methyl, oder Phenyl mono- oder disubstituierten Thiazol-2-ylrest oder einen unsubstituierten oder in l-Stellung durch $C_1$-$C_4$-Alkyl, wie Methyl, bzw.

Phenyl-$C_1$-$C_4$-alkyl, wie Benzyl, monosubstituierten Imidazol-2-ylrest bedeutet und $R_2$ für Wasserstoff steht, und ihre Salze, insbesondere ihre inneren Salze und pharmazeutisch verwendbaren Salze mit Basen.

Die Erfindung betrifft namentlich die in den Beispielen genannten Verbindungen der Formel I und ihre Salze, insbesondere ihre inneren Salze und pharmazeutisch verwendbaren Salze mit Basen.

Die Erfindung betrifft ferner ein auf an sich bekannten Methoden beruhendes Verfahren zur Herstellung von Verbindungen der Formel I und ihrer Salze. Dieses ist dadurch gekennzeichnet, dass man

a) in einer an einem substituierbaren N-Atom des Restes $R_1$ gegebenenfalls intermediär geschützten Verbindung der Formel

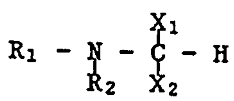

(II),

worin $X_1$ eine funktionell abgewandelte Phosphonogruppe X und $X_2$ Phosphono oder ebenfalls eine funktionell abgewandelte Phosphonogruppe X bedeutet, die Gruppe(n) X in freies Phosphono überführt oder

b) eine an einem substituierbaren N-Atom des Restes $R_1$ gegebenenfalls intermediär geschützte Verbindung der Formel

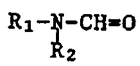

(III)

zunächst mit Phosphortrioxid und anschliessend mit Wasser umsetzt und gewünschtenfalls jeweils eine erhaltene Verbindung in eine andere Verbindung der Formel I und/oder eine erhaltene freie Verbindung in ein Salz oder ein erhaltenes Salz in die freie Verbindung oder in ein anderes Salz überführt.

Gemäss der Verfahrensvariante a) in Phosphono zu überführende funktionell abgewandelte Phosphonogruppen X liegen beispielsweise in einer Esterform, insbesondere einer Diesterform der Formel

$$-P(=O)(OR)_2 \quad (IIa)$$

vor, worin OR beispielsweise Niederalkoxy oder eine gegebenenfalls durch Niederalkyl, Niederalkoxy, Halogen, Trifluormethyl und/oder Hydroxy substituierte Phenyloxygruppe bedeutet.

Die Ueberführung einer funktionell abgewandelten in die freie Phosphonogruppe erfolgt in üblicher Weise durch Hydrolyse, beispielsweise in Gegenwart einer Mineralsäure, wie Chlor- bzw. Bromwasserstoffsäure oder Schwefelsäure, oder durch Umsetzung mit einem Triniederalkyl-halogensilan, z.B. mit Trimethylchlorsilan oder insbesondere Trimethyljodsilan oder Trimethylbromsilan, vorzugsweise unter Kühlen, z.B. im Temperaturbereich von etwa 0° bis etwa 25°C.

Die Ausgangsstoffe der Formel II können beispielsweise hergestellt werden, indem man eine Verbindung der Formel

$$R_1-N(R_2)-H \qquad (IIb;\ R_2=H)$$

mit der mindestens äguimolekularen Menge eines Orthoameisensäuretriesters der Formel

$$H-C(OR)_3 \qquad (IIc),$$

worin OR beispielsweise Niederalkoxy oder eine gegebenenfalls durch Niederalkyl, Niederalkoxy, Halogen, Trifluormethyl und/oder Hydroxy substituierte Phenyloxygruppe bedeutet, kondensiert, wobei primär wahrscheinlich eine entsprechende Verbindung der Formel

$$R_1-NH-CH(OR)_2\ (IId1)\ \text{oder}\ R_1-N=CH-OR\ (IId2)$$

gebildet wird, und das Kondensationsprodukt mit der mindestens doppeltmolaren Menge eines Phosphorigsäurediesters, z.B. der Formel

$$H-\underset{\underset{O}{\|}}{P}(OR)_2 \qquad (IIe),$$

weiterumsetzt und die erhaltene Verbindung (II, $R_2$=H) gewünschtenfalls zur entsprechenden Verbindung (II ; $R_2$=Niederalkyl) niederalkyliert.

In Zwischenprodukten II, worin der Rest $R_1$ durch Niederalkyl oder unsubstituiertes oder durch Niederalkyl, Niederalkoxy und/oder Halogen substituiertes Phenylniederalkyl N-substituiert ist, kann man den N-Substituenten abspalten, Niederalkyl beispielsweise durch Behandeln mit einem Halogenameisensäureester, wie einem Brom- oder Chlorameisensäureniederalkylester, und anschliessende Hydrolyse des gebildeten Carbamates und α-Phenylniederalkylreste beispielsweise durch Hydrogenolyse, z.B. Behandeln mit Wasserstoff in Gegenwart eines Hydrierungs-Katalysators, z.B. von Palladium auf Kohle oder/und Platinoxid, oder durch metallische Reduktion, z.B. Behandlung mit einem Alkalimetall in Ammoniak.

Man kann den Ausgangsstoff IIb aber auch in an sich bekannter Weise in Gegenwart eines Orthoameisensäuretriesters IIc mit dem Phosphorigsäurediester IIe umsetzen, ohne die Zwischenstufe zu isolieren. So setzt man gemäss einer besonders bevorzugten Ausführungsform in der Siedehitze die entsprechende Verbindung IIb in Gegenwart der mindestens äguimolekularen Menge eines Orthoameisensäuretriesters IIc mit der mindest doppeltmolaren Menge des Phosphorigsäurediesters IIe um, ohne die Zwischenstufe, z.B. der Formel IId1 bzw. IId2, zu isolieren, und hydrolysiert das Primärprodukt II durch Behandeln mit wässriger Salzsäure in der Siedehitze.

Die Umsetzung von Verbindungen III mit Phosphortrioxid gemäss Verfahrensvariante b) erfolgt vorzugsweise unter in-situ-Bildung des letzteren, beispielsweise durch Umsetzung von Phosphortrichlorid und phosphoriger Säure bei erhöhter Temperatur, z.B. bei etwa 50 bis 65°C, Hinzufügen der Reaktionskomponente III, weiteres Erwärmen und hydrolytische Aufarbeitung des Primärproduktes, eines 1 : 1-Adduktes des Aldehydes der Formel

$$R_1-\underset{\underset{R_2}{|}}{N}-CH=O \qquad (III)$$

mit Phosphortrioxid bislang unbekannter Struktur, vorzugsweise durch Behandeln mit Wasser.

In einer Abwandlung dieser bevorzugten Ausführungsform der Verfahrensvariante b) setzt man bei etwa 50°C bis 70°C Orthophosphorsäure mit einem etwa 1,1- bis etwa 2-fachen, vorzugsweise etwa 1,5-fachen, Ueberschuss an Phosphortrichlorid um, fügt die Reaktionskomponente III hinzu, erwärmt längere Zeit auf etwa 50°C bis 70°C, verdünnt mit 80%-iger Phosphorsäure und arbeitet hydrolytisch auf.

Ausgangsstoffe III können in üblicher Weise hergestellt werden, beispielsweise durch Umsetzung eines Amins der Formel

$R_1-N(R_2)-H$ (IIb; R=H)

mit Ameisensäure oder einem funktionellem Carboxyderivat davon, z.B mit einem Ameisensäureester der Formel

H-COOR (IIe),

worin OR beispielsweise eine gegebenenfalls durch Niederalkyl, Niederalkoxy, Halogen, Trifluormethyl und/oder Hydroxy substituierte Phenyloxygruppe bedeutet, oder mit Formamid

Für den intermediären Schutz eines substituierbaren N-Atoms des Restes $R_1$ eignen sich die üblichen N-Schutzgruppen und Einführungsund Abspaltungsverfahren derselben, beispielsweise 2,2,2-Trihalogen-, wie 2,2,2-Trijod-, 2,2,2-Tribrom- oder 2,2,2-Trichloräthoxycarbonylreste, die z.B. durch Behandeln mit Zink in Essigsäure abgespalten werden können, α-Phenylniederalkoxycarbonylreste, wie

Carbobenzoxy, die z.B. durch Katalytische Hydrierung abgespalten werden können, sowie Niederalkansulfonylgruppen, wie Methansulfonyl, die z.B. durch Behandeln mit Bis(2-Methoxyäthoxy)-natriumaluminiumhydrid abgespalten werden können, ebenso aber auch α-Phenylalkyloder Alkylgruppen, deren Abspaltung nachstehend behandelt wird.

Verfahrensgemäss oder nach einem anderen an sich bekannten Verfahren erhaltene Verbindungen der Formel I können in an sich bekannter Weise in andere Verbindungen der Formel I überführt werden.

So kann man Verbindungen der Formel I, worin $R_2$ Wasserstoff bedeutet, durch Umsetzung mit einem reaktiven Ester, wie einem Halogenwasserstoffsäureester oder organischen Sulfonsäureester, eines Niederalkanols Niederalkyl $R_2$ einführen. Man kann aber auch durch Umsetzung mit einem aliphatischen Aldehyd, z.B. mit Formaldehyd und Ameisensäure, einen aliphatischen Rest, z.B. Methyl, einführen.

Ferner kann man in Verbindungen der Formel I, worin der Rest $R_1$ durch Niederalkyl oder unsubstituiertes oder durch Niederalkyl, Niederalkoxy und/oder Halogen substituiertes Phenylniederalkyl N-substituiert ist, den N-Substituenten abspalten, Niederalkyl beispielsweise durch Behandeln mit einem Halogenameisensäureester, wie einem Brom- oder Chlorameisensäureniederalkylester, und anschliessende Hydrolyse des gebildeten Carbamates und α-PhenylNiederalkylreste beispielsweise durch Hydrogenolyse, z.B. Behandeln mit Wasserstoff in Gegenwart eines Hydrierungs-Katalysators, z.B. von Palladium auf Kohle oder/und Platinoxid oder durch metallische Reduktion, z.B. Behandlung mit einem Alkalimetall in Ammoniak.

Erhaltene freie Verbindungen der Formel I einschliesslich ihrer inneren Salze der Formel I′ können durch partielle oder vollständige Neutralisation mit einer der eingangs genannten Basen in Basesalze überführt werden. In analoger Weise kann man auch Säureadditionssalze der Formel I″ in die entsprechenden freien Verbindungen der Formel I bzw. inneren Salze der Formel I′ überführen.

Umgekehrt kann man erhaltene freie Verbindungen der Formel I durch Behandlung mit einer der eingangs genannten Protonensäuren in Säureadditionssalze der Formel I″ überführen.

Erhaltene Salze können in an sich bekannter Weise in die freien Verbindungen umgewandelt werden, z.B. durch Behandeln mit einem sauren Reagens, wie einer Mineralsäure, bzw. einer Base, z.B. Alkalilauge.

Die Verbindungen einschliesslich ihrer Salze können auch in Form ihrer Hydrate erhalten werden oder das zur Krisallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn-und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindungen ausgeht und die fehlenden Schritte durchführt oder einen Ausgangsstoff in Form eines Salzes und/oder Racemates bzw. Antipoden verwendet oder insbesondere unter den Reaktionsbedingungen bildet.

Die Erfindung betrifft ebenfalls bekannte Verbindungen der Formel I, worin $R_1$ einen unsubstituierten oder durch Niederalkyl und/oder Halogen mono- oder disubstituierten Pyrazol-3-ylrest oder Isoxazol3-ylrest bedeutet und $R_2$ Wasserstoff darstellt, namentlich 1-(Pyrazol-3-ylamino)methan-1,1-diphosphonsäure, 1-(4-Methylpyrazol-3-ylamino)methan-1,1-diphosphonsäure, 1-(5-Methylpyrazol-3-ylamino)methan-1,1-diphosphonsäure, 1-(Isoxazol-3-ylamino)methan-1,1-diphosphonsäure, 1-(4-Methylisoxazol-3-ylamino)methan-1,1-diphosphonsäure oder 1-(5-Methylisoxazol-3-ylamino)methan-1,1-diphosphonsäure, oder jeweils ein pharmazeutisch verwendbares Salz davon zur Anwendung in einem Verfahren zur Behandlung des menschlichen oder tierischen Körpers bzw. der Verwendung zur Herstellung

eines den Calciumstoffwechsel regulierenden Arzneimittels.

Die Erfindung betrifft weiterhin pharmazeutische Präparate enthaltend eine Verbindung der Formel I einschliesslich der vorstehend als bekannt bezeichneten oder ein pharmazeutisch verwendbares Salz davon.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu deren Herstellung bilden ebenfalls einen Gegenstand der Erfindung.

Bei den erfindungsgemässen pharmazeutischen Präparaten, welche Verbindungen der Formel I oder pharmazeutisch verwendbare Salze davon enthalten, handelt es sich um solche zur enteralen, wie oralen oder rektalen, und parenteralen Verabreichung, welche den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab. Im Normalfall ist für einen etwa 75 kg schweren Warmblüter bei oraler Applikation eine ungefähre Tagesdosis von etwa 20 bis 1000 mg, vorzugsweise etwa 30 bis 300 mg, bzw. bei intravenöser Verabreichung etwa 1 bis 25 mg, vorzugsweise etwa 1 bis 10 mg, vorteilhaft in mehreren gleichen Teildosen verteilt, zu veranschlagen.

Die neuen pharmazeutischen Präparate enthalten z.B. von etwa 10% bis etwa 80%, vorzugsweise von etwa 20% bis etwa 60%, des Wirkstoffs. Erfindungsgemässe pharmazeutische Präparate zur enteralen bzw. parenteralen Verabreichung sind z.B. solche in Dosiseinheitsformen wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragéee-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. vom Mais-, Weizen-, Reis- oder Kartoffelstärke, Gelatine, Tragaganth, Methylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten, Ueberzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit

Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enthalten ; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die vorliegende Erfindung betrifft ebenfalls die Verwendung der Verbindungen der Formel I und ihrer Salze, vorzugsweise zur Behandlung von auf Störungen des Calciumstoffwechsels zurückzuführenden Erkrankungen, z.B. des rheumatischen Formenkreises, und besonders von Osteoporosen.

Dosierungen unter 0,01 mg/kg Körpergewicht beeinflussen die pathologische Verkalkung bzw. die Auflösung von harten Geweben nur unerheblich. Bei Dosierungen von über 100 mg/kg Körpergewicht können langfristig toxische Nebenwirkungen auftreten. Die Verbindungen der Formel I und ihre Salze können sowohl oral als auch in hypertonischer Lösung subkutan, intramuskulär oder intravenös appliziert werden. Die bevorzugten Tagesdosen für diese Anwendungen liegen bei oraler Anwendung im Bereich von etwa 0,1 bis 5 mg/kg, bei subkutaner und intramuskulärer Applikation im Bereich von etwa 0,1 bis 1 mg/kg und bei intravenöser Applikation im Bereich von etwa 0,01 bis 2 mg/kg.

Die Dosierung der verwendeten Verbindungen ist jedoch variabel und hängt von den jeweiligen Konditionen wie Art und Schwere der Erkrankung, Dauer der Behandlung und der jeweiligen Verbindung ab. Einzeldosen enthalten beispielsweise von 0,01 bis 10 mg, Dosiseinheitsformen für parenterale, wie intravenöse Applikation z.B. von 0,01 bis 0,1 mg, vorzugsweise 0,02 bis 0,08 mg, orale Dosiseinheitsformen z.B. von 0,2 bis 2,5 mg, vorzugsweise 0,3 bis 1,5 mg pro kg Körpergewicht. Die bevorzugte Einzeldosierung beträgt bei oraler Applikation 10 bis 100 mg und bei intravenöser Applikation 0,5 bis 5 mg und kann in bis zu 4 Einzeldosen täglich verabreicht werden. Die höheren Dosierungen bei oraler Applikation sind infolge der begrenzten Resorption erforderlich. Bei längerdauernden Behandlungen kann nach anfänglich höherer Dosierung normalerweise auf niedrige Dosierungen umgestellt werden, um den gewünschten Effekt aufrechtzuerhalten.

Die nachfolgenden Beispiele illustrieren die oben beschriebene Erfindung ; sie sollen jedoch diese in ihrem Umfang in keiner Weise einschränken. Temperaturen sind in Celsiusgraden angegeben.

Beispiel 1 : 6,57 g (17 mMol) 1-(Thiazol-2-ylamino)methan-1,1-diphosphonsäuretetraäthylester werden in 70 ml n-Salzsäure gelöst und 6 Stunden zum Rückfluss erhitzt. Im Verlaufe der Reaktion fällt das Produkt in Form eines feinen weissen Niederschlags aus. Nach Kühlen auf Raumtemperatur wird filtriert und mit wässrigem Methanol gewaschen. Man erhält 4,33 g (93% d.Th.) 1-(Thiazol-2-ylamino)-methan-1,1-diphosphonsäure vom Smp. 275° (Zers.).

Das Ausgangsmaterial kann z.B. folgendermassen hergestellt werden :

Eine Mischung, bestehend aus 10.0 g (0.1 Mol) 2-Aminothiazol, 20.0 ml (0.12 Mol) Orthoameisensäuretriäthylester und 26.6 ml (0.2 Mol) Diäthylphosphit wird 1 Stunde zum Rückfluss erhitzt. Das freigesetzte Aethanol wird abdestilliert, wobei die Innentemperatur allmählich auf etwa 150° ansteigt. Der Rückstand wird in Chloroform aufgenommen und über Kieselgel filtriert. Das Rohprodukt wird durch Säulenchromatographie (Kieselgel/Essigester) gereinigt. Man erhält 4.37 g (11% d.Th.) 1-(Thiazol-2-ylamino)methan-1,1-diphosphonsäuretetraäthylester vom Smp. 103 - 104°.

Beispiel 2 : In analoger Weise wie in Beispiel 1 beschrieben kann man auch 1-(Oxazol-2-ylamino)methan-1,1-diphosphonsäure, Smp. 245° (Zers.) und 1-(Benzoxazol-2-ylamino)methan-1,1-diphosphonsäure, Smp. 270° (Zers.), herstellen.

Beispiel 3 : 4,36 g (10 mMol) 1-(Benzthiazol-2-ylamino)methan-1,1-diphosphonsäure-tetraäthylester werden in 40 ml n-Salzsäure 6 Stunden auf 110°-120° erhitzt. Im Verlaufe der Reaktion fällt das Produkt in Form eines weissen Niederschlags aus. Nach Kühlen auf Raumtemperatur wird filtriert und mit wässrigem Methanol gewaschen. Man erhält 3,09 g (95% d.Th.) 1-(Benzthiazol-2-ylamino)methan-1,1-diphosphonsäure vom Smp. 290° (Zers.).

Das Ausgangsmaterial kann z.B. folgendermassen hergestellt werden :

Eine Mischung, bestehend aus 3,0 g (20 mMol) 2-Aminobenzthiazol, 4,0 ml (24 mMol) Orthoameisensäuretriäthylester und 5,3 ml (40 mMol) Diäthylphosphit wird 5 Stunden auf 120°-125° erhitzt. Der zu Beginn der Reaktion ausfallende gelbe Niederschlag geht allmählich wieder in Lösung. Das freigesetzte Aethanol wird während der Reaktion abdestilliert. Das beim Stehenlassen fest werdende Rohprodukt wird durch Säulenchromatographie (Kieselgel/Essigester/Methanol) gereinigt. Man erhält 5,62 g (64% d.Th.) 1-(Benzthiazol-2-ylamino)-methan-1,1-diphosphonsäure-tetraäthylester vom Smp. 165-167°.

Beispiel 4 : 1,30 g (3,2 mMol) 1-(4-Methylthiazol-2-ylamino)methan-1,1-diphosphonsäure-tetraäthylester werden in 20 ml In-Salzsäure während 20 h auf 100° erhitzt. Nach dem Abkühlen wurden 20 ml Methanol zugegeben. Während des anschliessenden Rührens fällt das Produkt in Form feiner weisser Kristalle aus. Das Filtrat wird mit Methanol und Petroläther nachgewaschen. Ausbeute : 615 mg (67% d.Th.) 1-(4-Methylthiazol-2-ylamino)methan-1,1-diphosphonsäure vom Smp. 294° (Zers.).

Das Ausgangsmaterial kann z.B. folgendermassen hergestellt werden :

Eine Mischung, bestehend aus 2,33 g (20 mMol) 2-Amino-4-methylthiazol, 4,0 ml (24 mMol) Orthoameisensäuretriäthylester und 5,3 ml (40 mMol) Diäthylphosphit wird 4 Stunden auf 120-125° erhitzt. Das

freigesetzte Aethanol wird abdestilliert. Der Rückstand wird durch Säulenchromatographie (Kieselgel/Essigester/Methanol) gereinigt. Man erhält 1,32 g (17% d.Th.) 1-(4-Methylthiazol-2-ylamino)methan-1,1-diphosphonsäure-tetraäthylester in Form eines viskosen Oels.

Beispiel 5 : 1,97 g (4,9 mMol) 1-(5-Methylthiazol-2-ylamino)methan-1,1-diphosphonsäure-tetraäthylester werden in 20 ml n-Salzsäure 6 Stunden zum Rückfluss erhitzt. Beim Abkühlen und Stehenlassen bei Raumtemperatur kristallisiert das Produkt. Es wird filtriert und mit Aceton und Petroläther gewaschen. Ausbeute : 0,64 g (45% d.Th.) 1-(5-Methylthiazol-2-ylamino)methan-1,1-diphosphonsäure vom Smp. 208° (Zers.).

Das Ausgangsmaterial kann z.B. folgendermassen hergestellt werden :

Eine Mischung, bestehend aus 1,14 g (10 mMol) 2-Amino-5-methylthiazol, 2,0 ml (12 mMol) Orthoameisensäuretriäthylester und 2,65 ml (20 mMol) Diäthylphosphit wird 4 $\frac{1}{2}$ Stunden auf 120-125° erhitzt. Das freigesetzte Aethanol wird abdestilliert. Der Rückstand wird durch Säulenchromatographie (Kieselgel/Essigester/Methanol) gereinigt. Man erhält 1,97 g (49% d.Th.) 1-(5-Methylthiazol-2-ylamino)methan-1,1-diphosphonsäure-tetraäthylester in Form eines viskosen Oels.

Beispiel 6 : 4,02 g (8,7 mMol) 1-(5-Phenylthiazol-2-ylamino)methan-1,1-diphosphonsäuretetraäthylester werden in 30 ml n-Salzsäure 18 Stunden zum Rückfluss erhitzt. Nach Abkühlen auf Raumtemperatur wird etwas Methanol zugegeben und filtriert. Das Filtrat wird 1 Stunde in Methanol zum Rückfluss erhitzt, heiss filtriert und 2-mal mit heissem Methanol gewaschen. Ausbeute : 2,90 g (95% d.Th.) 1-(5-Phenylthiazol-2-ylamino)methan-1,1-diphosphonsäure vom Smp. 290° (Zers.).

Das Ausgangsmaterial kann z.B. folgendermassen hergestellt werden :

Eine Mischung, bestehend aus 2,93 g (16,6 mMol) 2-Amino-5-phenylthiazol, 3,3 ml (19,9 mMol) Orthoameisensäuretriäthylester und 4,4 ml (33,5 mMol) Diäthylphosphit wird zunächst während 2 Stunden auf 120°, dann 2 Stunden auf 130° erhitzt. Freigesetztes Aethanol wird im Verlaufe der Reaktion abdestilliert. Das sich beim Abkühlen verfestigende Produkt wird durch Chromatographie (Kieselgel/Essigester/Methanol) gereinigt. Man erhält 4,12 g (54% d.Th) 1-(5-Phenylthiazol-2-ylamino)methan-1,1-diphosphonsäuretetraäthylester vom Smp. 151-153°.

Beispiel 7 : 2,5 g (5,96 mMol) 1-(Benzimidazol-2-ylamino)methan-1,1-diphosphonsäuretetraäthylester werden in 25 ml ln-Salzsäure gelöst und 26 Stunden auf 100-110° erwärmt. Im Verlauf der Reaktion fällt das Produkt in Form eines feinen, weissen Niederschlags aus. Es wird heiss filtriert und mit Wasser und anschliessend mit Methanol gewaschen. Man erhält 0,23 g (13% d.Th.) 1-(1-Benzimidazol-2-ylamino)methan-1,1-diphosphonsäure vom Smp. 265° (Zers.).

Das Ausgangsmaterial kann z.B. folgendermassen hergestellt werden.

6,66 g (50 mMol) 2-Aminobenzimidazol, 10,0 ml (60 mMol) Orthoameisensäuretriäthylester und 13,3 ml (101 mMol) Diäthylphosphit werden vermischt und 2 Stunden bei 125-130° gerührt, bis kein Aethanol mehr abdestilliert. Der Rückstand wird durch Säulenchromatographie (Kieselgel/Essigester/Methanol, 9 : 1) gereinigt. Man erhält 2,89 g (14% d.Th.) 1-(Benzimidazol-2-ylamino)methan-1,1-diphosphonsäuretetraäthylester vom Smp. 169-170°.

Beispiel 8 : 7 g Phosphortrichlorid werden mit 4,0 g phosphoriger Säure vermischt und eine Stunde unter Rühren auf 60° erwärmt. Man fügt 6,12 g N-(Thiazol-2-yl)formamid hinzu und erwärmt weitere 6 Stunden auf etwa 60°. Dann verrührt man mit 30 ml Wasser, saugt ab, wäscht mit wässrigem Methanol nach und trocknet unter vermindertem Druck. Man erhält 2,0 g 1-(Thiazol-2-ylamino)methan-1,1-diphosphonsäure vom Smp. 275° (Zers.).

Beispiel 9 : 2,0 g (20,4 mMol) kristalline Orthophosphorsäure werden mit 3,5 g (25,5 mMol) Phosphortrichlorid 1 Stunde bei 55-60° gerührt. Danach gibt man 4,08 g (20,0 mMol) N-(4-Phenylthiazol-2-yl)formamid hinzu. Die Reaktionsmischung wird etwa 24 Stunden bei 60° stehengelassen. Zur Verdünnung gibt man 10 ml 80%-ige Phosphorsäure 80%ig hinzu und lässt über Nacht bei Raumtemperatur stehen. Darauf erhitzt man erneut auf 60-70° und gibt weitere 1,37 g (10 mMol) Phosphortrichlorid hinzu, lässt 2 Stunden 60-70° weiterrühren, fügt 30 ml Wasser und 20 ml Aceton hinzu und lässt 2 Stunden bei 60° ausrühren. Man lässt auf Raumtemperatur abkühlen, filtriert den feinen, blassgelben Niederschlag ab und wäscht mit Wasser/Aceton 3 : 2. Der Rückstand wird gereinigt, indem man 1- mal mit Wasser/Aceton 1 : 1 und 2- mal mit Methanol auskocht. Man erhält 180 mg (2,6% d.Th.) 1-(4-phenylthiazol-2-ylamino)methan-1-diphosphonsäure vom Smp. 298° (Zers.)

Das Ausgangsmaterial kann folgendermassen hergestellt werden :

13,22 g (75 mMol) 2-Amino-4-phenylthiazol werden 5 Stunden mit 40 ml Ameisensäure auf 110° erwärmt. Man lässt auf Raumtemperatur abkühlen und giesst auf Eis. Es fällt ein weisser Niederschlag aus, der abfiltriert und mit Eiswasser nachgewaschen wird. Man kristallisiert aus petrolether um und erhält 7,01 g (45,8% d.Th) 4-(phenylthiazol-2-amino)formamid vom Smp. 161-164°.

Beispiel 10 : In an sich bekannter Weise, z.B. wie in den Beispielen 1 bis 7 beschrieben, kann man

ferner herstellen :

1-(Imidazol-2-ylamino)methan-1,1-diphosphonsäure,
1-(Imidazol-4-ylamino)methan-1,1-diphosphonsäure,
1-(1-Methylimidazol-2-ylamino)methan-1,1-diphosphonsäure,
1-(Tetrazol-5-ylamino)methan-1,1-diphosphonsäure,
1-(Oxazol-2-ylamino)methan-1,1-diphosphonsäure,
1-(1,3,4-Thiadiazol-2-ylamino)methan-1,1-diphosphonsäure,
1-(5-Methyl-1,3,4-thiadiazol-2-ylamino)methan-1,1-diphosphonsäure, Smp. 260° (Zers.),
1-(3-phenyl-1,2,4-thiadiazol-5-ylamino)methan-1,1-diphosphonsäure, Smp. 198°.

Beispiel 11 : Tabletten, enthaltend 50 mg Wirkstoff, z.B. 1-(Thiazol-2-ylamino)methan-1,1-diphosphonsäure oder ein Salz, z.B. das Natriumsalz, davon, können folgendermassen hergestellt werden :

| Bestandteile (für 1000 Tabletten) | |
|---|---|
| Wirkstoff | 50,0 g |
| Lactose | 50,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung : Sämtliche festen Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des polyäthylenglykols in 100 ml Wasser hinzugegeben. Der erhaltene Kleister wird zu der pulvermischung hinzugefügt und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 12 : Tabletten, enthaltend 100 mg Wirkstoff, z.B. 1-(Thiazol-2-ylamino)methan-1,1-diphosphonsäure oder ein Salz, z.B. das Natriumsalz, davon können folgendermassen hergestellt werden :

| Bestandteile (für 1000 Tabletten) | |
|---|---|
| Wirkstoff | 100,0 g |
| Lactose | 100,0 g |
| Weizenstärke | 47,0 g |
| Magnesiumstearat | 3,0 g |

Herstellung : Sämtliche festen Ingredienzien werden zunächst durch ein Sieb mit 0,6 mm Maschenweite getrieben. Dann wird der Wirkstoff, die Lactose, das Magnesiumstearat und die Hälfte der Stärke vermischt. Die andere Hälfte der Stärke wird in 40 ml Wasser suspendiert und diese Suspension zu 100 ml siedendem Wasser hinzugegeben. Der erhaltene Kleister wird zu der pulvermischung hinzugefügt und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 13 : In analoger Weise wie in den Beispielen 11 und 12 beschrieben können auch Tabletten, enthaltend jeweils 100 mg bzw. 50 mg einer anderen der in den Beispielen 1 bis 10 genannten Verbindungen der Formel I hergestellt werden, wobei diese auch in Form von Salzen mit Basen, z.B. als Dinatriumsalz, vorliegen können.

Beispiel 14 : Kautabletten, enthaltend 75 mg Wirkstoff, z.B. 1-(Thiazol-2-ylamino)methan-1,1-diphosphonsäure oder ein Salz, z.B. das Natriumsalz davon, können z.B. folgendermassen hergestellt werden :

| Zusammensetzung: (für 1000 Tabletten) | |
|---|---|
| Wirkstoff | 75,0 g |
| Mannit | 230,0 g |
| Lactose | 150,0 g |
| Talkum | 21,0 g |
| Glycin | 12,5 g |
| Stearinsäure | 10,0 g |
| Saccharin | 1,5 g |
| 5 %-ige Gelatinelösung | q.s. |

Herstellung : Alle festen Ingredienzien werden zunächst durch ein Sieb mit 0,25 mm Maschenweite getrieben. Der Mannit und die Lactose werden gemischt, unter Hinzufügen von Gelatinelösung granuliert, durch ein Sieb mit 2 mm Maschenweite getrieben, bei 50° getrocknet und nochmals durch ein Sieb mit 1,7 mm Maschenweite getrieben. Der Wirkstoff, das Glycin und das Saccharin werden sorgfältig vermischt, der Mannit, das Lactosegranulat, die Stearinsäure und das Talkum hinzugegeben, das Ganze gründlich vermischt und zu beidseitig konkaven Tabletten von etwa 10 mm Durchmesser mit Bruchrille auf der Oberseite verpresst.

In analoger Weise können auch Tabletten, enthaltend jeweils 75 mg einer anderen der in den Beispielen 1 bis 10 genannten Verbindungen der Formel I hergestellt werden, wobei diese auch in Form von Salzen mit Basen, z.B. als Dinatriumsalz, vorliegen können.

Beispiel 15 : Tabletten, enthaltend 10 mg Wirkstoff, z.B. 1-(Thiazol-2-ylamino)methan-1,1-diphosphonsäure oder ein Salz, z.B. das Natriumsalz davon, können folgendermassen hergestellt werden :

| Zusammensetzung (für 1000 Tabletten) | |
|---|---|
| Wirkstoff | 10,0 g |
| Lactose | 328,5 g |
| Maisstärke | 17,5 g |
| Polyäthylenglykol 6000 | 5,0 g |
| Talkum | 25,0 g |
| Magnesiumstearat | 4,0 g |
| entmineralisiertes Wasser | q.s. |

Herstellung : Die festen Ingredienzien werden zunächst durch ein Sieb mit 0,6 mm Maschenweite getrieben. Dann werden Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des polyäthylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpresst.

In analoger Weise können auch Tabletten, enthaltend 10 mg einer anderen Verbindung der Formel I gemäss den Beispielen 1 bis 10 hergestellt werden, wobei diese auch in Form von Salzen mit Basen, z.B. als Dinatriumsalz, vorliegen können.

Beispiel 16 : Gelatinesteckkapseln, enthaltend 100 mg Wirkstoff, z.B. 1-(Thiazol-2-ylamino)methan-1,1-diphosphonsäure oder ein Salz, z.B. das Natriumsalz davon, können folgendermassen hergestellt werden :

| Zusammensetzung (für 1000 Kapseln) | |
|---|---|
| Wirkstoff | 350,0 g |
| mikrokristalline Cellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem Wirkstoff (lyophilisiert) gesiebt und beide Komponenten 10 Minuten lang innig vermischt. Dann wird die mikrokristalline Cellulose durch ein Sieb mit einer Maschenweite von 0,9 mm hinzugesiebt und erneut 10 Minuten innig vermischt. Zuletzt wird das Magnesiumstearat durch ein Sieb mit einer Maschenweite von 0,8 mm hinzugesiebt und nach 3 Minuten weiteren Mischens je 390 mg der Mischung in Gelatinesteckkapseln der Grösse O (elongated) abgefüllt.

In analoger Weise können auch Kapseln, enthaltend 100 mg einer anderen Verbindung der Formel I gemäss den Beispielen 1 bis 10 hergestellt werden, wobei diese auch in Form von Salzen mit Basen, z.B. als Dinatriumsalz, vorliegen können.

Beispiel 17 : Eine 0,2%ige Injektions- bzw. Infusionslösung kann beispielsweise folgendermassen hergestellt werden.

| | |
|---|---|
| Wirkstoff, z.B. 1-(Thiazol-2-ylamino)methan-1,1-diphosphonsäure oder ein Salz, z.B. das Natriumsalz davon | 5,0 g |
| Natriumchlorid | 22,5 g |
| Phosphatpuffer pH = 7,4 | 300,0 g |
| Wasser, entmin. | ad 2500,0 ml |

Der Wirkstoff wird in 1000 ml Wasser gelöst und durch ein Mikrofilter filtriert. Man versetzt mit der pufferlösung und füllt mit Wasser auf 2500 ml auf. Zur Herstellung von Dosiseinheitsformen werden je 1,0 oder 2,5 ml in Glasampullen (enthaltend je 2,0 bzw. 5,0 mg Wirkstoff abgefüllt).

## Ansprüche

### Patentansprüche (für die Vertragsstaaten BE, CH, DE, FR, GB, IT, LI, LU, NL, SE)

1. Heteroarylaminomethandiphosphonsäuren der Formel

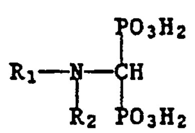

(I)

worin $R_1$ einen C-unsubstituierten oder durch Niederalkyl, durch Niederalkoxy, durch unsubstituiertes oder durch Niederalkyl, Niederalkoxy und/oder Halogen mono- oder disubstituiertes Phenyl, durch Hydroxy, durch Diniederalkylamino, durch Niederalkylthio und/oder durch Halogen C-mono- oder C-disubstituierten und/oder an einem gegebenenfalls vorhandenen substituierbaren N-Atom unsubstituiertes oder vorzugsweise durch Niederalkyl oder durch unsubstituiertes oder durch Niederalkyl, Niederalkoxy und/oder Halogen mono- oder disubstituiertes Phenylniederalkyl N-monosubstituierten Imidazolyl-, Benzimidazolyl-, 2H-1,2,3- oder 4H-1,2,4-Triazolyl-, Tetrazolyl-, Oxazolyl-, Benzoxazolyl-, Oxadiazolyl-, Thiazolyl-, Benzthiazolyl-oder Thiadiazolylrest bedeutet und $R_2$ für Wasserstoff oder Niederalkyl steht, wobei unter niederen Resten solche zu verstehen sind, die bis und mit 7C-Atome aufweisen, und ihre Salze.

2. Verbindungen der Formel I, gemäss Anspruch 1, worin $R_1$ einen unsubstituierten oder durch $C_1$-$C_4$-Alkyl oder durch einen unsubstituierten oder durch $C_1$-$C_4$-Alkyl, $C_1$-$C_4$-Alkoxy und/oder Halogen mono- oder

disubstituierten Phenylrest C-substituierten Thiazolylrest, Benzthiazol-2-ylrest, Thiadiazolylrest, Oxazolylrest oder Benzoxazol-2-ylrest oder durch einen $C_1$-$C_4$-Alkyl oder durch einen unsubstituierten oder durch $C_1$-$C_4$-Alkyl, $C_1$-$C_4$-Alkoxy und/oder Halogen mono- oder disubstituierten Phenyl-$C_1$-$C_4$-alkylrest N-substituierten Imidazolylrest oder Benzimidazol-2-ylrest bedeutet und $R_2$ für Wasserstoff steht, und ihre Salze.

3.Verbindungen der Formel I S.27, worin $R_1$ einen unsubstituierten oder durch $C_1$-$C_4$-Alkyl, $C_1$-$C_4$-Alkoxy, Phenyl, Hydroxy, Di-$C_1$-$C_4$-Alkyl-amino, $C_1$-$C_4$-Alkylthio oder Halogen der Atomnummer bis und mit 35, substituieren Thiazolylrest bedeutet und $R_2$ für Wasserstoff steht, und ihre Salze.

4. Verbindungen der Formel I S.27, worin $R_1$ einen unsubstituierten oder, insbesondere in 4- und/oder 5-Stellung, durch $C_1$-$C_4$-Alkyl oder Phenyl mono- oder disubstituierten Thiazol-2-ylrest, oder einen unsubstituiereten oder in 1-Stellung durch $C_1$-$C_4$-Alkyl bzw. Phenyl-$C_1$-$C_4$-alkyl monosubstituierten Imidazol-2-ylrest bedeutet und $R_2$ für Wasserstoff steht, und ihre Salze.

5. 1-(Thiazol-2-ylamino)methan-1,1-diphosphonsäure gemäss Anspruch 1 oder eine Salz davon.

6. 1-(5-Methyl-1,3,4,-thiadiazol-2-ylamino)methan-1,1-diphosphonsäure gemäss Anspruch 1 oder ein Salz davon.

7. 1-(1,3,4-Thiadiazol-2-ylamino)methan-1,1-diphosphonsäure oder ein Salz davon.

8. 1-(1-Methylimidazol-2-ylamino)methan-1,1-diphosphonsäure gemäss Anspruch 1 oder ein Salz davon.

9. 1-(Oxazol-2-ylamino)methan-1,1-diphosphonsäure gemäss Anspruch 1 oder ein Salz davon.

10. 1-(3-Phenyl-1,2,4-thiadiazol-5-ylamino)methan-1,1-diphosphonsäure gemäss Anspruch 1 oder ein Salz davon.

11. 1-(Benzthiazol-2-ylamino)methan-1,1-diphosphonsäure gemäss Anspruch 1 oder ein Salz davon.

12. 1-(Benzoxazol-2-ylamino)methan-1,1-diphosphonsäure gemäss Anspruch 1 oder ein Salz davon.

13. 1-(4-Methylthiazol-2-ylamino)methan-1,1-diphosphonsäure gemäss Anspruch 1 oder ein Salz davon.

14. 1-(5-Methylthiazol-2-ylamino)methano-1,1-diphosphonsäure gemäss Anspruch 1 oder ein Salz davon.

15. Eine Verbindung gemäss einem der Ansprüche 1, 2, 4, 6-8, 10, 13 und 14 zur therapeutischen Behandlung des menschlichen oder tierischen Körpers.

16. Eine Verbindung gemäss einem der Ansprüche 3, 4, 5 und 9-12 zur therapeutischen Behandlung des menschlichen oder tierischen Körpers.

17. Pharmazeutische Präparate, enthaltend eine Verbindung der Formel

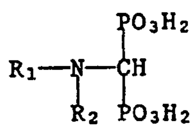 (I)

worin $R_1$ einen C-unsubstituierten oder durch Niederalkyl, durch Niederalkoxy, durch unsubstituiertes oder durch Niederalkyl, Niederalkoxy und/oder Halogen mono- oder disubstituiertes Phenyl, durch Hydroxy, durch Diniederalkylamino, durch Niederalkylthio und/oder durch Halogen C-mono- oder C-disubstituierten und/oder an einem gegebenenfalls vorhandenen substituierbaren N-Atom unsubstituiertes oder vorzugsweise durch Niederalkyl oder durch unsubstituiertes oder durch Niederalkyl, Niederalkoxy und/oder Halogen mono- oder disubstituiertes Phenylniederalkyl N-monosubstituierten Imidazolyl-, Benzimidazolyl-, 2H-1,2,3- oder 4H-1,2,4-Triazolyl-, Tetrazolyl-, Oxazolyl-, Benzoxazolyl-, Oxadiazolyl-, Thiazolyl-, Benzthiazolyl-oder Thiadiazolylrest bedeutet und $R_2$ für Wasserstoff oder Niederalkyl steht, oder worin $R_1$ einen unsubstituierten oder durch Niederalkyl, unsubstituiertes oder durch Niederalkyl und/oder Halogen mono- oder disubstituierten Pyrazol-3-ylrest oder Isoxazol-3-ylrest darstellt und $R_2$ Wasserstoff bedeutet, S.27 oder ein pharmazeutische verwendbares Salz davon neben üblichen pharmazeutischen Hilfsstoffen.

18. Pharmazeutische Präparate, enthaltend eine Verbindung gemäss einem der Ansprüche 1, 2, 4, 6-8, 10 und 13-15 neben üblichen pharmazeutischen Hilfsstoffen.

19. Pharmazeutische Präparate, enthaltend eine Verbindung gemäss einem der Ansprüche 3, 4, 9-12 und 16 neben üblichen pharmazeutischen Hilfsstoffen.

20. Pharmazeutische Präparate gemäss Anspruch 17, enthaltend 1-(Pyrazol-3-ylamino)-methan-1,1-diphosphonsäure, 1-(4-Methylpyrazol-3-ylamino)methan-1,1-diphosphonsäure, 1-(5-Methylpyrazol-3-ylamino)-methan-1,1-diphosphonsäure, 1-(Isoxazol-3-ylamino)methan-1,1-diphosphonsäure, 1-(4-Methylisoxazol-3-ylamino)methan-1,1-diphosphonsäure oder 1-(5-Methylisoxazol-3-ylamino)methan-1,1-diphosphonsäure, oder jeweils ein pharmazeutisch verwendbares Salz davon neben üblichen pharmazeutischen Hilfsstoffen.

21. Verfahren zur Herstellung von Heteroarylaminomethandiphosphonsäuren der Formel

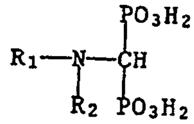 (I)

worin $R_1$ einen C-unsubstituierten oder durch Niederalkyl, durch Niederalkoxy, durch unsubstituiertes oder durch Niederalkyl, Niederalkoxy und/oder Halogen mono- oder disubstituiertes Phenyl, durch Hydroxy, durch Diniederalkylamino, durch Niederalkylthio und/oder durch Halogen C-mono- oder C-disubstituierten und/oder an einem gegebenenfalls vorhandenen substituierbaren N-Atom unsubstituiertes oder vorzugsweise durch Niederalkyl oder durch unsubstituiertes oder durch Niederalkyl, Niederalkoxy und/oder Halogen mono- oder disubstituiertes Phenylniederalkyl N-monosubstituierten Imidazolyl-, Benzimidazolyl-, 2H-1,2,3- oder 4H-1,2,4-Triazolyl-, Tetrazolyl-, Oxazolyl-, Benzoxazolyl-, Oxadiazolyl-, Thiazolyl-, Benzthiazolyl-oder Thiadiazolylrest bedeutet und $R_2$ für Wasserstoff oder Niederalkyl steht, S.27 und ihrer Salze, dadurch gekennzeichnet, dass man

a) in einer an einem substituierbaren N-Atom des Restes $R_1$ gegebenenfalls intermediär geschützten Verbindung der Formel

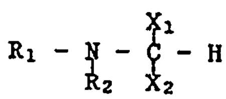 (II),

worin $X_1$ eine funktionell abgewandelte Phosphonogruppe X und $X_2$ Phosphono oder ebenfalls eine funktionell abgewandelte Phosphonogruppe X bedeutet, die Gruppe(n) X in freies Phosphono überführt oder oder

b) eine an einem substituierbaren N-Atom des Restes $R_1$ gegebenenfalls intermediär geschützte Verbindung der Formel

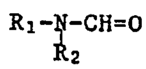 (III)

zunächst mit Phosphortrioxid und anschliessend mit Wasser umsetzt und gewünschtenfalls jeweils eine erhaltene Verbindung in eine andere Verbindung der Formel I und/oder eine erhaltene freie Verbindung in ein Salz oder ein erhaltenes Salz in die freie Verbindung oder in ein anderes Salz überführt.

22. Verwendung von Verbindungen gemäss einem der Ansprüche 1-16 zur Herstellung von den Calciumstoffwechsel regulierenden pharmazeutischen Präparaten.

**Patentansprüche für die Vertragsstaaten : AT, ES, GR**

1. Verfahren zur Herstellung von Heteroarylaminomethandiphosphonsäuren der Formel

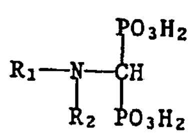 (I)

worin $R_1$ einen C-unsubstituierten oder durch Niederalkyl, durch Niederalkoxy, durch unsubstituiertes oder durch Niederalkyl, Niederalkoxy und/oder Halogen mono- oder disubstituiertes Phenyl, durch Hydroxy, durch Diniederalkylamino, durch Niederalkylthio und/oder durch Halogen C-mono- oder C-disubstituierten und/oder an einem gegebenenfalls vorhandenen substituierbaren N-Atom unsubstituiertes oder vorzugsweise durch Niederalkyl oder durch unsubstituiertes oder durch Niederalkyl, Niederalkoxy und/oder Halogen mono- oder disubstituiertes Phenylniederalkyl N-monosubstituierten Imidazolyl-, Benzimidazolyl-, 2H-1,2,3- oder 4H-1,2,4-Triazolyl-, Tetrazolyl-, Oxazolyl-, Benzoxazolyl-, Oxadiazolyl-, Thiazolyl-, Benzthiazolyl-oder Thiadiazolylrest bedeutet und $R_2$ für Wasserstoff oder Niederalkyl steht, S.27 und ihrer Salze, dadurch gekennzeichnet, dass man

a) in einer an einem substituierbaren N-Atom des Restes $R_1$ gegebenenfalls intermediär geschützten Verbindung der Formel

$$R_1 - \underset{R_2}{\underset{|}{N}} - \overset{X_1}{\overset{|}{\underset{X_2}{\underset{|}{C}}}} - H \qquad (II),$$

worin $X_1$ eine funktionell abgewandelte Phosphonogruppe X und $X_2$ Phosphono oder ebenfalls eine funktionell abgewandelte Phosphonogruppe X bedeutet, die Gruppe(n) X in freies Phosphono überführt oder

b) eine an einem substituierbaren N-Atom des Restes $R_1$ gegebenenfalls intermediär geschützte Verbindung der Formel

$$R_1-\underset{R_2}{\underset{|}{N}}-CH{=}O \qquad (III)$$

zunächst mit Phosphortrioxid und anschliessend mit Wasser umsetzt und gewünschtenfalls jeweils eine erhaltene Verbindung in eine andere Verbindung der Formel I und/oder eine erhaltene freie Verbindung in ein Salz oder ein erhaltenes Salz in die freie Verbindung oder in ein anderes Salz überführt.

2. Verfahren gemäss Anspruch 1, dadurch gekennzeichnet dass Verbindungen der Formel I, worin $R_1$ einen unsubstituierten oder durch $C_1$-$C_4$-Alkyl oder durch einen unsubstituierten oder durch $C_1$-$C_4$ Alkyl, $C_1$-$C_4$-Alkoxy und/oder Halogen mono- oder disubstituierten Phenylrest C-substituierten Thiazolylrest, Benzthiazol-2-ylrest, Thiadiazolylrest, Oxazolylrest oder Benzoxazol-2-ylrest oder durch einen $C_1$-$C_4$-Alkyl oder durch einen unsubstituierten oder durch $C_1$-$C_4$-Alkyl, $C_1$-$C_4$-Alkoxy und/oder Halogen mono-oder disubstituierten Phenyl-$C_1$-$C_4$-alkylrest N-substituierten Imidazol-2-ylrest oder Benzimidazol-2-ylrest bedeutet und $R_2$ für Wasserstoff steht, oder ihre Salze herstellt.

3. Verfahren gemäss Anspruch 1, dadurch gekennzeichnet dass Verbindungen der Formel I, worin $R_1$ einen unsubstituierten oder durch $C_1$-$C_4$-Alkyl, $C_1$-$C_4$-Alkoxy, Phenyl, Hydroxy, Di-$C_1$-$C_4$-Alkylamino, $C_1$-$C_4$-Alkylthio oder Halogen der Atomnummer bis und mit 35 substituierten Thiazolylrest bedeutet und $R_2$ für Wasserstoff steht, oder ihre Salze herstellt.

4. Verfahren gemäss Anspruch 1, dadurch gekennzeichnet dass Verbindungen der Formel I, worin $R_1$ einen unsubstituierten oder, insbesondere in 4- und/oder 5-Stellung, durch $C_1$-$C_4$-Alkyl oder Phenyl mono- oder disubstituierten Thiazol-2-ylrest, einen unsubstituierten oder in 1-Stellung durch $C_1$-$C_4$-Alkyl bzw. Phenyl-$C_1$-$C_4$-alkyl monosubstituierte Imidazol-2-ylrest bedeutet und $R_2$ für Wasserstoff steht, oder ihre Salze herstellt.

5. Verfahren gemäss Anspruch 1, dadurch gekennzeichnet dass man 1-(Thiazol-2-ylamino)methan-1,1-diphosphonsäure oder ein Salz davon herstellt.

6. Verfahren gemäss Anspruch 1, dadurch gekennzeichnet dass man 1-(5-Methyl-1,3,4-thiadiazol-2-ylamino)methan-1,1-diphosphonsäure oder ein Salz davon herstellt.

7. Verfahren gemäss Anspruch 1, dadurch gekennzeichnet dass man 1-(1,3,4-Thiadiazol-2-ylamino)methan-1,1-diphosphonsäure oder ein Salz davon herstellt.

8. Verfahren gemäss Anspruch 1, dadurch gekennzeichnet dass man 1-(1-Methylimidazol-2-ylamino)methan-1,1-diphosphonsäure oder ein Salz davon herstellt.

9. Verfahren gemäss Anspruch 1, dadurch gekennzeichnet dass man 1-(Oxazol-2-ylamino)methan-1,1-diphosphonsäure oder ein Salz davon herstellt.

10. Verfahren gemäss Anspruch 1, dadurch gekennzeichnet dass 1-(3-Phenyl-1,2,4-thiadiazol-5-ylamino)methan-1,1-diphosphonsäure oder ein Salz davon herstellt.

11. Verfahren gemäss Anspruch 1, dadurch gekennzeichnet dass man 1-(Benzthiazol-2-ylamino)methan-1,1-diphosphonsäure oder ein Salz davon herstellt.

12. Verfahren gemäss Anspruch 1, dadurch gekennzeichnet dass man 1-(Benzoxazol-2-ylamino)methan-1,1-diphosphonsäure oder ein Salz davon herstellt.

13. Verfahren gemäss Anspruch 1, dadurch gekennzeichnet dass 1-(4-Methylthiazol-2-ylamino)methan-1,1-diphosphonsäure oder ein Salz davon herstellt.

14. Verfahren gemäss Anspruch 1, dadurch gekennzeichnet dass man 1-(5-Methylthiazol-2-ylamino)methan-1,1-diphosphonsäure oder ein Salz davon herstellt.

15. Verfahren zur Herstellung von pharmazeutischen Präparaten, dadurch gekennzeichnet, dass man eine Verbindung der Formel

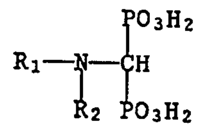

(I)

worin $R_1$ einen C-unsubstituierten oder durch Niederalkyl, durch Niederalkoxy, durch unsubstituiertes oder durch Niederalkyl, Niederalkoxy undloder Halogen mono- oder disubstituiertes Phenyl, durch Hydroxy, durch Diniederalkylamino, durch Niederalkylthio und/oder durch Halogen C-mono- oder C-disubstituierten und/oder an einem gegebenenfalls vorhandenen substituierbaren N-Atom unsubstituiertes oder vorzugsweise durch Niederalkyl oder durch unsubstituiertes oder durch Niederalkyl, Niederalkoxy und/oder Halogen mono- oder disubstituiertes Phenylniederalkyl N-monosubstituierten Imidazolyl-, Benzimidazolyl-, 2H-1,2,3- oder 4H-1,2,4-Triazolyl-, Tetrazolyl-, Oxazolyl-, Benzoxazolyl-, Oxadiazolyl-, Thiazolyl-, Benzthiazolyl-oder Thiadiazolylrest bedeutet und $R_2$ für Wasserstoff oder Niederalkyl steht, oder worin $R_1$ einen unsubstituierten oder durch Niederalkyl, unsubstituiertes oder durch Niederalkyl und/oder Halogen mono- oder disubstituierten Pyrazol-3-ylrest oder Isoxazol-3-ylrest darstellt und $R_2$ Wasserstoff bedeutet, S.27 oder ein pharmazeutisch verwendbares Salz davon mit üblichen pharmazeutischen Hilfsstoffen vermischt.

16. Verfahren zur Herstellung von pharmazeutischen Präparaten, dadurch gekennzeichnet, dass man eine Verbindung, erhältlich gemäss einem der Ansprüche 1-14 mit üblichen pharmazeutischen Hilfsstoffen vermischt.

17. Verfahren gemäss Anspruch 15 zur Herstellung von pharmazeutischen Präparaten, enthaltend 1-(Pyrazol-3-ylamino)methan-1,1diphosphonsäure, 1-(4-Methylpyrazol-3-ylamino)methan-1,1-diphosphonsäure, 1-(5-Methylpyrazol-3-ylamino)-methan-1,1-diphosphonsäure, 1-(Isoxazol-3-ylamino)methan-1,1-diphosphonsäure, 1-(4-Methylisoxazol-3-ylamino)methan-1,1-diphosphonsäure oder 1-(5-Methylisoxazol-3-ylamino)methan-1,1-diphosphonsäure, oder jeweils ein pharmazeutisch verwendbares Salz davon.

## Claims

### Claims for the Contracting States BE, CH, DE, GB, IT, LI, LU, FR, NL and SE.

1. Heteroarylaminomethanediphosphonic acids of the formula

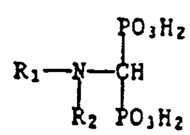

(I)

in which $R_1$ represents an imidazolyl, benzimidazolyl, 2H1,2,3- or 4H-1,2,4-triazolyl, tetrazolyl, oxazolyl, benzoxazolyl, oxadiazolyl, thiazolyl, benzothiazolyl or thiadiazolyl radical that is C-unsubstituted or C-mono- or C-di-substituted by lower alkyl ; by lower alkoxy ; by phenyl that is unsubstituted or is mono- or di-substituted by lower alkyl, lower alkoxy and/or by halogen ; by hydroxy ; by di-lower alkylamino ; by lower alkylthio and/or by halogen ; and/or that is unsubstituted at a substitutable N-atom which may optionally be present or preferably N-mono-substituted by lower alkyl or by phenyl-lower alkyl that is unsubstituted or is mono- or di-substituted by lower alkyl, lower alkoxy and/or by halogen ; and $R_2$ represents hydrogen or lower alkyl ; lower radicals being intended to mean those having up to and including 7 carbon atoms, and their salts.

2. Compounds of the formula I according to claim 1, in which $R_1$ represents a thiazolyl radical, a benzothiazol2-yl radical, a thiadiazolyl radical, an oxazolyl radical or a benzoxazol-2-yl radical each of which is unsubstituted or is C-substituted by $C_1$-$C_4$alkyl or by a phenyl radical that is unsubstituted or is mono- or di-substituted by $C_1$-$C_4$alkyl, $C_1$-$C_4$alkoxy and/or by halogen ; or represents an imidazol-2-yl radical or a ben-

zimidazol-2yl radical each of which is N-substituted by $C_1$-$C_4$alkyl or by a phenyl-$C_1$-$C_4$alkyl radical that is unsubstituted or is mono- or di-substituted by $C_1$-$C_4$alkyl, $C_1$-$C_4$alkoxy and/or by halogen ; and $R_2$ represents hydrogen, and their salts.

3. Compounds of the formula I according to claim 1, in which $R_1$ represents a thiazolyl radical that is unsubstituted or is substituted by $C_1$-$C_4$alkyl, $C_1$-$C_4$alkoxy, phenyl, hydroxy, di-$C_1$-$C_4$alkylamino, $C_1$-$C_4$alkylthio or by halogen having an atomic number of up to and including 35, and $R_2$ represents hydrogen, and their salts.

4. Compounds of the formula I according to claim 1, in which $R_1$ represents a thiazol-2-yl radical that is unsubstituted or is mono- or di-substituted, especially in the 4- and/or 5-position, by $C_1$-$C_4$alkyl or by phenyl, or represents an imidazol-2-yl radical that is unsubstituted or is mono-substituted in the 1-position by $C_1$-$C_4$alkyl or by phenyl-$C_1$-$C_4$alkyl, and $R_2$ represents hydrogen, and their salts.

5. 1-(thiazol-2-ylamino)methane-1,1-diphosphonic acid according to claim 1 or a salt thereof.

6 1-(5-methyl-1,3,4-thiadiazol-2-ylamino)methane-1,1diphosphonic acid according to claim 1 or a salt thereof.

7. 1-(1,3,4-thiadiazol-2-ylamino)methane-1,1-diphosphonic acid or a salt thereof.

8. 1-(1-methylimidazo1-2-ylamino)methane-1,1-diphosphonic acid according to claim 1 or a salt thereof.

9. 1-(oxazol-2-ylamino)methane-1,1-diphosphonic acid according to claim 1 or a salt thereof.

10. 1-(3-phenyl-1,2,4-thiadiazol-5-ylamino)methane-1,1diphosphonic acid according to claim 1 or a salt thereof.

11. 1-(benzothiazol-2-ylamino)methane-1,1-diphosphonic acid according to claim 1 or a salt thereof.

12. 1-(benzoxazol-2-ylamino)methane-1,1-diphosphonic acid according to claim 1 or a salt thereof.

13. 1-(4-methylthiazol-2-ylamino)methane-1,1-diphosphonic acid according to claim 1 or a salt thereof.

14. 1-(5-methylthiazol-2-ylamino)methano-1,1-diphosphonic acid according to claim 1 or a salt thereof.

15. A compound according to any one of claims 1, 2, 4, 6 to 8, 10, 13 and 14 for the therapeutic treatment of the human or animal body.

16. A compound according to any one of claims 3, 5, 9, 11 and 12 for the therapeutic treatment of the human or animal body.

17. Pharmaceutical preparations containing a compound of the formula

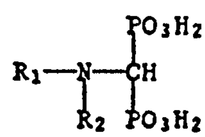

(I)

in which $R_1$ represents an imidazolyl, benzimidazolyl, 2H1,2,3- or 4H-1,2,4-triazolyl, tetrazolyl, oxazolyl, benzoxazolyl, oxadiazolyl, thiazolyl, benzothiazolyl or thiadiazolyl radical that is C-unsubstituted or C-mono- or C-di-substituted by lower alkyl ; by lower alkoxy ; by phenyl that is unsubstituted or is mono- or di-substituted by lower alkyl, lower alkoxy and/or by halogen ; by hydroxy ; by di-lower alkylamino ; by lower alkylthio and/or by halogen ; and/or that is unsubstituted at a substitutable N-atom which may optionally be present or preferably N-mono-substituted by lower alkyl or by phenyl-lower alkyl that is unsubstituted or is mono- or di-substituted by lower alkyl, lower alkoxy and/or by halogen ; and $R_2$ represents hydrogen or lower alkyl, or in which $R_1$ represents a pyrazol-3-yl radical or an isoxazol-3-yl radical each of which is unsubstituted or is mono- or di-substituted by lower alkyl and/or halogen, and $R_2$ represents hydrogen, lower radicals being intended to mean those having up to and including 7 carbon atoms, or a pharmaceutically acceptable salt thereof, together with customary pharmaceutical adjuncts.

18. Pharmaceutical preparations containing a compound according to any one of claims 1, 2, 4, 6 to 8, 10 and 13 to 15, together with customary pharmaceutical adjuncts.

19. Pharmaceutical preparations containing a compound according to any one of claims 3, 5, 9, 11, 12 and 16, together with customary pharmaceutical adjuncts.

20. Pharmaceutical preparations according to claim 17, containing 1-(pyrazol-3-ylamino)methane-1,1-diphosphonic acid, 1-(4-methylpyrazol-3-ylamino)methane-1,1-diphosphonic acid, 1-(5-methylpyrazol-3-ylamino)methane-1,1-diphosphonic acid, 1-(isoxazol-3-ylamino)methane-1,1-diphosphonic acid, 1-(4-methylisoxazol-3-ylamino)methane-1,1-diphosphonic acid or 1-(5-methylisoxazol-3-ylamino)-methane-1,1-diphosphonic acid, or in each case a pharmaceutically acceptable salt thereof, together with customary pharmaceutical adjuncts.

21. A process for the preparation of heteroarylaminomethanediphosphonic acids of the formula

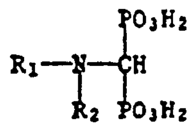

(I)

in which $R_1$ represents an imidazolyl, benzimidazolyl, 2H1,2,3- or 4H-1,2,4-triazolyl, tetrazolyl, oxazolyl, benzoxazolyl, oxadiazolyl, thiazolyl, benzothiazolyl or thiadiazolyl radical that is C-unsubstituted or C-mono- or C-di-substituted by lower alkyl ; by lower alkoxy ; by phenyl that is unsubstituted or is mono- or di-substituted by lower alkyl, lower alkoxy and/or by halogen ; by hydroxy ; by di-lower alkylamino ; by lower alkylthio and/or by halogen ; and/or that is unsubstituted at a substitutable N-atom which may optionally be present or preferably N-mono-substituted by lower alkyl or by phenyl-lower alkyl that is unsubstituted or is mono- or di-substituted by lower alkyl, lower alkoxy and/or by halogen ; and $R_2$ represents hydrogen or lower alkyl, lower radicals being intended to mean those having up to and including 7 carbon atoms, and their salts, characterised in that

a) in a compound of the formula

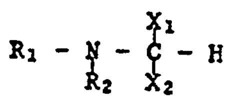

(II)

which is optionally intermediately protected at a substitutable N-atom of the radical $R_1$ and in which $X_1$ represents a functionally modified phosphono group X and $X_2$ represents phosphono or similarly represents a functionally modified phosphono group X, the group(s) X is(are) converted into free phosphono, or

b) a compound of the formula

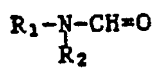

(III)

which is optionally intermediately protected at a substitutable N-atom of the radical $R_1$, is reacted first with phosphorus trioxide and then with water, and, if desired, in each case, a resulting compound is converted into a different compound of formula I and/or a resulting free compound is converted into a salt or a resulting salt is converted into the free compound or into a different salt.

22. The use of compounds according to any one of claims 1 to 16 for the manufacture of pharmaceutical preparations that regulate the calcium metabolism.

**Claims for the Contracting States AT, ES, GR.**

1. A process for the preparation of heteroarylaminomethanediphosphonic acids of the formula

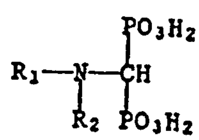

(I)

in which $R_1$ represents an imidazolyl, benzimidazolyl, 2H1,2,3- or 4H-1,2,4-triazolyl, tetrazolyl, oxazolyl, benzoxazolyl, oxadiazolyl, thiazolyl, benzothiazolyl or thiadiazolyl radical that is C-unsubstituted or C-mono- or C-di-substituted by lower alkyl ; by lower alkoxy ; by phenyl that is unsubstituted or is mono- or di-substituted by lower alkyl, lower alkoxy and/or by halogen ; by hydroxy ; by di-lower alkylamino ; by lower alkylthio and/or by halogen ; and/or that is unsubstituted at a substitutable N-atom which may optionally be present or preferably N-mono-substituted by lower alkyl or by phenyl-lower alkyl that is unsubstituted or is mono- or di-substituted by lower alkyl, lower alkoxy and/or by halogen ; and $R_2$ represents hydrogen or lower alkyl, lower radicals being intended to mean those having up to and including 7 carbon atoms, and their salts, characterised in that

a) in a compound of the formula

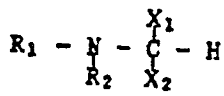 (II)

which is optionally intermediately protected at a substitutable N-atom of the radical $R_1$ and in which $X_1$ represents a functionally modified phosphono group X and $X_2$ represents phosphono or similarly represents a functionally modified phosphono group X, the group(s) X is(are) converted into free phosphono, or

b) a compound of the formula

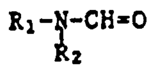 (III)

which is optionally intermediately protected at a substitutable N-atom of the radical $R_1$, is reacted first with phosphorus trioxide and then with water, and, if desired, in each case, a resulting compound is converted into a different compound of formula I and/or a resulting free compound is converted into a salt or a resulting salt is converted into the free compound or into a different salt.

2. A process according to claim 1, characterised in that compounds of the formula I in which $R_1$ represents a thiazolyl radical, a benzothiazol-2-yl radical, a thiadiazolyl radical, an oxazolyl radical or a benzoxazol-2-yl radical each of which is unsubstituted or is Csubstituted by $C_1$-$C_4$alkyl or by a phenyl radical that is unsubstituted or is mono- or di-substituted by $C_1$-$C_4$ alkyl, $C_1$-$C_4$alkoxy and/or by halogen ; or represents imidazol-2-yl radical or benzimidazol-2-yl radical each of which is N-substituted by a $C_1$-$C_4$alkyl or by a phenyl$C_1$-$C_4$alkyl radical that is unsubstituted or is mono- or di-substituted by $C_1$-$C_4$alkyl, $C_1$-$C_4$alkoxy and/or by halogen ; and $R_2$ represents hydrogen, or their salts are prepared.

3. A process according to claim 1, characterised in that compounds of the formula I in which $R_1$ represents a thiazolyl radical that is unsubstituted or is substituted by $C_1$-$C_4$alkyl, $C_1$-$C_4$alkoxy, phenyl, hydroxy, di-$C_1$-$C_4$ alkylamino, $C_1$-$C_4$alkylthio or by halogen having an atomic number of up to and including 35, and $R_2$ represents hydrogen, or their salts are prepared.

4. A process according to claim 1, characterised in that compounds of the formula I in which $R_1$ represents a thiazol-2-yl radical that is unsubstituted or is mono- or di-substituted, especially in the 4- and/or 5-position, by $C_1$-$C_4$alkyl or by phenyl, or represents an imidazol-2-yl radical that is unsubstituted or is mono-substituted in the 1-position by $C_1$-$C_4$alkyl or by phenyl-$C_1$-$C_4$alkyl, and $R_2$ represents hydrogen, or their salts are prepared.

5. A process according to claim 1, characterised in that 1-(thiazol-2-ylamino)methane-1,1-diphosphonic acid or a salt thereof is prepared.

6. A process according to claim 1, characterised in that 1-(5-methyl-1,3,4-thiadiazol-2-ylamino)methane-1,1-diphosphonic acid or a salt thereof is prepared.

7. A process according to claim 1, characterised in that 1-(1,3,4-thiadiazol-2-ylamino)methane-1,1-diphosphonic acid or a salt thereof is prepared.

8. A process according to claim 1, characterised in that 1-(1-methylimidazol-2-ylamino)methane-1,1-diphosphonic acid or a salt thereof is prepared.

9. A process according to claim 1, characterised in that 1-(oxazol-2-ylamino)methane-1,1-diphosphonic acid or a salt thereof is prepared.

10. A process according to claim 1, characterised in that 1-(3-phenyl-1,2,4-thiadiazol-5-ylamino)methane-1,1-diphosphonic acid or a,salt thereof is prepared.

11. A process according to claim 1, characterised in that 1-(benzothiazol-2-ylamino)methane-1,1-diphosphonic acid or a salt thereof is prepared.

12. A process according to claim 1, characterised in that 1-(benzoxazol-2-ylamino)methane-1,1-diphosphonic acid or a salt thereof is prepared.

13. A process according to claim 1, characterised in that 1-(4-methylthiazol-2-ylamino)methane-1,1-diphosphonic acid or a salt thereof is prepared.

14. A process according to claim 1, characterised in that 1-(5-methylthiazol-2-ylamino)methane-1,1-diphosphonic acid or a salt thereof is prepared.

15. A process for the manufacture of pharmaceutical preparations, characterised in that a compound of the formula

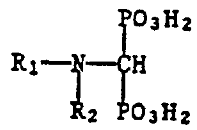

(I)

in which $R_1$ represents an imidazolyl, benzimidazolyl, 2H1,2,3- or 4H-1,2,4-triazolyl, tetrazolyl, oxazolyl, benzoxazolyl, oxadiazolyl, thiazolyl, benzothiazolyl or thiadiazolyl radical that is C-unsubstituted or C-mono- or C-di-substituted by lower alkyl ; by lower alkoxy ; by phenyl that is unsubstituted or is mono- or di-substituted by lower alkyl, lower alkoxy and/or by halogen ; by hydroxy ; by di-lower alkylamino ; by lower alkylthio and/or by halogen ; and/or that is unsubstituted at a substitutable N-atom which may optionally be present or preferably N-mono-substituted by lower alkyl or by phenyl-lower alkyl that is unsubstituted or is mono- or di-substituted by lower alkyl, lower alkoxy and/or by halogen ; and $R_2$ represents hydrogen or lower alkyl, or in which $R_1$ represents a pyrazol-3-yl radical or an isoxazol-3-yl radical each of which is unsubstituted or is mono- or di-substituted by lower alkyl and/or halogen, and $R_2$ represents hydrogen, lower radicals being intended to mean those having up to and including 7 carbon atoms, or a pharmaceutically acceptable salt thereof, is mixed with customary pharmaceutical adjuncts.

16. A process for the manufacture of pharmaceutical preparations, characterised in that a compound obtainable in accordance with any one of claims 1 to 14 is mixed with customary pharmaceutical adjuncts.

17. A process according to claim 15 for the manufacture of pharmaceutical preparations containing 1-(pyrazol-3-ylamino)methane-1,1-diphosphonic acid, 1-(4-methylpyrazol-3-ylamino)methane-1,1-diphosphonic acid, 1-(5methylpyrazol-3-ylamino)methane-1,1-diphosphonic acid, 1-(isoxazol-3-ylamino)methane-1,1-diphosphonic acid, 1-(4methylisoxazol-3-ylamino)methane-1,1-diphosphonic acid or 1-(5-methylisoxazol-3-ylamino)methane-1,1-diphosphonic acid, or in each case a pharmaceutically acceptable salt thereof.

## Revendications

### Revendications pour les Etats contractants : BE, CH, DE, GB, IT, LI, LU, FR, NL, SE.

1. Acides hétéroarylaminométhanediphosphoniques de formule

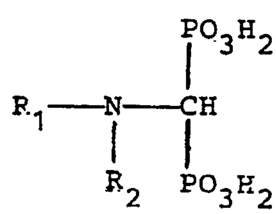

(I)

où $R_1$ représente les restes imidazolyle, benzimidazolyle, 2H-1,2,3- ou 4H-1,2,4-triazolyle, tétrazolyle, oxazolyle, benzoxazolyle, oxadiazolyle, thiazolyle, benzthiazolyle ou thiadiazolyle non substitués en C ou mono- ou disubstitués en C par un alkyle inférieur, un alcoxy inférieur, un phényle non substitué ou mono- ou disubstitué par un alkyle inférieur, par un alcoxy inférieur et/ou par un halogène, un hydroxy, un dialkyle inférieur amino, un alkyle inférieur thio et/ou un halogène et/ou monosubstitués en N sur un atome de N substituable éventuellement présent par un phénylalkvle inférieur non substitué ou de préférence mono- ou disubstitué par un alkyle inférieur, un alcoxy inférieur et/ou un halogène et $R_2$ représente l'hydrogène ou un alkyle inférieur, où par restes inférieurs, on entend des restes présentant jusqu'à 7 atomes de carbone, et leurs sels.

2. Composés de formule I selon la revendication 1 où $R_1$ représente un reste thiazolyle, un reste benzothiazol-2-yle, un reste thiadiazolyle, un reste oxazolyle ou un reste benzoxazol-2-yle non substitués ou substitués en C par un alkyle en $C_1$-$C_4$ ou par un reste phényle non substitué ou mono- ou disubstitué par un alkyle en $C_1$-$C_4$, par un alcoxy en $C_1$-$C_4$ et/ou par un halogène ou représente un reste imidazolyle ou un reste benzimidazol2-yle substitué en N par un alkyle en $C_1$-$C_4$ ou par un reste phénylalkyle en $C_1$-$C_4$ non substitué ou mono- ou disubstitué par un alkyle en $C_1$-$C_4$, par un alcoxy en $C_1$-$C_4$ et/ou par un halogène et $R_2$ représente l'hydrogène et leurs sels.

3. Composés de formule I selon la revendication 1 où $R_1$ représente un reste thiazolyle non substitué ou substitué par un alkyle en $C_1$-$C_4$, par un alcoxy en $C_1$-$C_4$, par un phényle, par l'hydroxy, par un dialkyle

en $C_1$-$C_4$ amino, par un alkyle en $C_1$-$C_4$ thio ou par un halogène de numéro atomique allant jusqu'à 35 et $R_2$ représente l'hydrogène et leurs sels.

4. Composés de formule I selon la revendication 1 où $R_1$ représente un reste thiazol-2-yle non substitué ou mono- ou disubstitué, en particulier en position 4 et/ou 5, par un alkyle en $C_1$-$C_4$ ou par un phényle ou un reste imidazol-2-yle non substitué ou monosubstitué en position 1 par un alkyle en $C_1$-$C_4$ ou par un phénylalkyle en $C_1$-$C_4$ et $R_2$ représente l'hydrogène et leurs sels.

5. L'acide 1-(thiazol-2-ylamino)méthane-1,1diphosphonique selon la revendication 1 ou l'un de ses sels.

6. L'acide 1-(5-méthyl-1,3,4-thiadiazol-2ylamino)méthane-1,1-diphosphonique selon la revendication 1 ou l'un de ses sels.

7. L'acide 1-(1,3,4-thiadiazol-2-ylamino) méthane-1,1-diphosphonique ou l'un de ses sels.

8. L'acide 1-(1-méthylimidazol-2-ylamino)méthane-1,1-diphosphonique selon la revendication 1 ou l'un de ses sels.

9. L'acide 1-(oxazol-2-ylamino)méthane-1,1-diphosphonique selon la revendication 1 ou l'un de ses sels.

10. L'acide 1-(3-phényl-1,2,4-thiadiazol-5ylamino)méthane-1,1-diphosphonique selon la revendication 1 ou l'un de ses sels.

11. L'acide 1-(benzothiazol-2-ylamino)méthane-1,1-diphosphonique selon la revendication 1 ou l'un de ses sels.

12. L'acide 1-(benzoxazol-2-ylamino)méthane-1,1-diphosphonique selon la revendication 1 ou l'un de ses sels.

13. L'acide 1-(4-méthylthiazol-2-ylamino)méthane-1,1-diphosphonique selon la revendication 1 ou l'un de ses sels.

14. L'acide 1-(5-méthylthiazol-2-ylamino)méthane-1,1-diphosphonique selon la revendication 1 ou l'un de ses sels.

15. Un composé selon l'une des revendications 1, 2, 4, 6-8, 10, 13 et 14 destiné au traitement thérapeutique du corps humain ou animal.

16. Un composé selon l'une des revendications 3, 4, 5 et 9-12 destiné au traitement thérapeutique du corps humain ou animal.

17. Préparations pharmaceutiques contenant un composé de formule

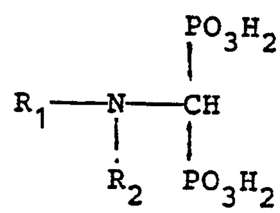

(I)

où $R_1$ représente les restes imidazolyle, benzimidazolyle, 2H-1, 2, 3- ou 4H-1, 2, 4-triazolyle, tétrazolyle, oxazolyle, benzoxazolyle, oxadiazolyle, thiazolyle, benzothiazolyle ou thiadiazolyle non substitués en C ou mono- ou disubstitués en C par un alkyle inférieur, un alcoxy inférieur, un phényle non substitué ou mono- ou disubstitué par un alkyle inférieur, par un alcoxy inférieur et/ou par un halogène, un hydroxy, un dialkyle inférieur amino, un alkyle inférieur thio et/ou un halogène et/ou monosubstitués en N sur un atome de N substituable éventuellement présent par un phénylalkyle inférieur non substitué ou de préférence mono- ou disubstitué par un alkyle inférieur, un alcoxy inférieur et/ou un halogène et $R_2$ représente l'hydrogène ou un alkyle inférieur ou où $R_1$ représente un reste pyrazol-3-yle ou isoxazol-3-yle non substitué ou mono- ou disubstitué par un alkyle inférieur et/ou par un halogène et $R_2$ représente l'hydrogène, où par restes inférieurs, on entend des restes présentant jusqu'à 7 atomes de carbone ou l'un de ses sels pharmaceutiquement utilisables à côté des adjuvants pharmaceutiques usuels.

18. Préparations pharmaceutiques contenant un composé selon l'une des revendications 1,2,4,6-8,10 et 13-15 à côté des adjuvants pharmaceutiques usuels.

19. Préparations pharmaceutiques contenant un composé selon l'une des revendications 3,4,9-12 et 16 à côté des adjuvants pharmaceutiques usuels.

20. Préparations pharmaceutiques selon la revendication 17 contenant l'acide 1-(pyrazol-3-ylamino)méthane-1,1-diphosphonique, l'acide 1-(4-méthylpyrazol-3-ylamino) méthane-1,1-diphosphonique, l'acide 1-(5-méthylpyrazol-3-ylamino)méthane-1,1-diphosphonique, l'acide 1-(isoxazol-3-ylamino)méthane-1,1-diphosphonique, l'acide 1-(4-méthylisoxazol-3-ylamino)méthane-1,1-diphosphonique ou l'acide 1-(5-méthylisoxazol-3-ylamino)méthane-1,1-diphosphonique ou l'un de leurs sels pharmaceutiquement utilisables à côté des adjuvants pharmaceutiques usuels.

21. Procédé pour la préparation des acides hétéroarylaminométhanediphosphoniques de formule

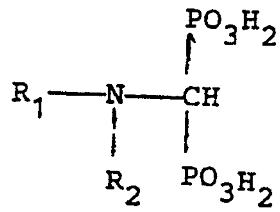

(I)

où $R_1$ représente les restes imidazolyle, benzimidazolyle, 2H-1,2,3- ou 4H-1,2,4-triazolyle, tétrazolyle, oxazolyle, benzoxazolyle, oxadiazolyle, thiazolyle, benzothiazolyle ou thiadiazolyle non substitués en C ou mono- ou disubstitués en C par un alkyle inférieur, un alcoxy inférieur, un phényle non substitué ou mono- ou disubstitué par un alkyle inférieur, par un alcoxy inférieur et/ou par un halogène, un hydroxy, un dialkyle inférieur amino, un alkyle inférieur thio et/ou un halogène et/ou monosubstitués en N sur un atome de N substituable éventuellement présent par un phénylalkyle inférieur non substitué ou de préférence mono- ou disubstitué par un alkyle inférieur, un alcoxy inférieur et/ou un halogène et $R_2$ représente l'hydrogène ou un alkyle inférieur et de leurs sels, caractérisé

a) en ce que l'on transforme, dans un composé éventuellement intermédiairement protégé sur un atome de N substituable du reste $R_1$, de formule

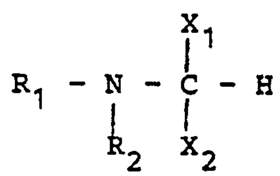

(II)

où $X_1$ représente un groupe phosphono X fonctionnellement modifié et $X_2$ un groupe phosphono ou éventuellement un groupe phosphono X fonctionnellement modifié, le ou les groupe(s) X en phosphono libre ou

b) en ce que l'on fait réagir un composé de formule

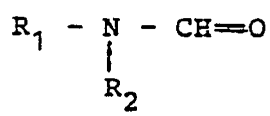

(III)

éventuellement intermédiairement protégé sur un atome de N substituable du reste $R_1$, d'abord, avec le trioxyde de phosphore puis avec l'eau et, si désiré, en ce que l'on transforme le composé obtenu en un autre composé de formule I et/ou le composé libre obtenu en un sel ou le sel obtenu en un composé libre ou en un autre sel.

22. Utilisation des composés selon l'une des revendications 1-16 pour l'obtention de préparations pharmaceutiques régulant le métabolisme du calcium.

**Revendications pour les Etats contractants : AT, ES, GR.**

1. Procédé pour la préparation des acides hétéroarylaminométhanediphosphoniques de formule

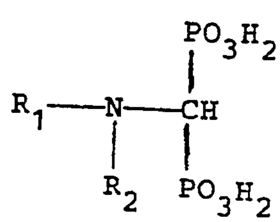

(I)

où $R_1$ représente les restes imidazolyle, benzimidazolyle, 2H-1, 2, 3- ou 4H-1, 2, 4-triazolyle, tétrazolyle, oxazolyle, benzoxazolyle, oxadiazolyle, thiazolyle, benzthiazolyle ou thiadiazolyle non substitués en C ou mono- ou disubstitués en C par un alkyle inférieur, un alcoxy inférieur, un phényle non substitué ou mono- ou disubstitué par un alkyle inférieur, par un alcoxy inférieur et/ou par un halogène, un hydroxy, un dialkyle inférieur amino, un alkyle inférieur thio et/ou un halogène et/ou monosubstitués en N sur un atome de N substituable éventuellement présent par un phénylalkyle inférieur non substitué ou de préférence mono- ou disubstitué par un alkyle inférieur, un alcoxy inférieur et/ou un halogène et $R_2$ représente l'hydrogène ou un alkyle inférieur, où par restes inférieurs, on entend des restes présentant, jusqu'à 7 atomes de carbone, et de leurs sels caractérisé

a) en ce que l'on transforme, dans un composé éventuellement intermédiairement protégé sur un atome de N substituable du reste $R_1$, de formule

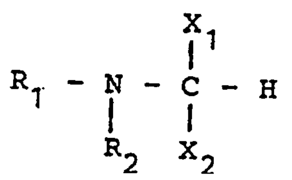

(II)

où $X_1$ représente un groupe phosphono X fonctionnellement modifié et $X_2$ un groupe phosphono ou éventuellement un groupe phosphono X fonctionnellement modifié, le ou les groupe(s) X en phosphono libre ou

b) en ce que l'on fait réagir un composé de formule

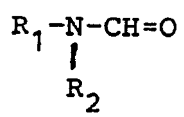

(III)

éventuellement intermédiairement protégé sur un atome de N substituable du reste $R_1$, d'abord, avec le trioxyde de phosphore puis avec l'eau et, si désiré, en ce que l'on transforme le composé obtenu en un autre composé de formule I et/ou le composé libre obtenu en un sel ou le sel obtenu en un composé libre ou en un autre sel.

2. Procédé selon la revendication 1, caractérisé en ce que l'on prépare les composés de formule I où $R_1$ représente un reste thiazolyle, un reste benzothiazol-2-yle, un reste thiadiazolyle, un reste oxazolyle ou un reste benzoxazol-2-yle non substitués ou substitués en C par un alkyle en $C_1$-$C_4$ ou par un reste phényle non substitué ou mono- ou disubstitué par un alkyle en $C_1$-$C_4$, par un alcoxy en $C_1$-$C_4$ et/ou par un halogène ou représente un reste imidazol-2-yle ou un reste benzimidazol-2-yle substitué en N par un alkyle en $C_1$-$C_4$ ou par un reste phénylalkyle en $C_1$-$C_4$ non substitué ou mono- ou disubstitué par un alkyle en $C_1$-$C_4$, par un alcoxy en $C_1$-$C_4$ et/ou par un halogène et $R_2$ représente l'hydrogène ou leurs sels.

3. Procédé selon la revendication 1, caractérisé en ce que l'on prépare les composés de formule I où $R_1$ représente un reste thiazolyle non substitué ou substitué par un alkyle en $C_1$-$C_4$, par un alcoxy en $C_1$-$C_4$, par un phényle, par l'hydroxy, par un dialkyle en $C_1$-$C_4$ amino, par un alkyle en $C_1$-$C_4$ thio ou par un halogène de numéro atomique allant jusqu'à 35 et $R_2$ représente l'hydrogène ou leurs sels.

4. Procédé selon la revendication 1, caractérisé en ce que l'on prépare les composés de formule I où $R_1$ représente un reste thiazol-2-yle non substitué ou mono- ou disubstitué, en particulier en position 4 et/ou 5, par un alkyle en $C_1$-$C_4$ ou par un phényle, un reste imidazol-2-yle non substitué ou monosubstitué en position 1 par un alkyle en $C_1$-$C_4$ ou par un phénylalkyle en $C_1$-$C_4$ et $R_2$ représente l'hydrogène ou leurs sels.

5. Procédé selon la revendication 1, caractérisé en ce que l'on prépare l'acide 1-(thiazol-2-ylamino)méthane-1,1-diphosphonique ou l'un de ses sels.

6. Procédé selon la revendication 1, caractérisé en ce que l'on prépare l'acide 1-(5-méthyl-1,3,4-thiadiazol-2-ylamino)méthane-1,1-diphosphonique ou l'un de ses sels.

7. Procédé selon la revendication 1, caractérisé en ce que l'on prépare l'acide 1-(1,3,4-thiadiazol-2-ylamino)méthane-1,1-diphosphonique ou l'un de ses sels.

8. Procédé selon la revendication 1, caractérisé en ce que l'on prépare l'acide 1-(1-méthylimidazol-2-ylamino)méthane-1,1-diphosphonique ou l'un de ses sels.

9. Procédé selon la revendication 1, caractérisé en ce que l'on prépare l'acide 1-(oxazol-2-ylamino)méthane-1,1-diphosphonique ou l'un de ses sels.

10. Procédé selon la revendication 1, caractérisé en ce que l'on prépare l'acide 1-(3-phényl -1,2,4-thiadiazol-5-ylamino)méthane-1,1-diphosphonique ou l'un de ses sel.

11. Procédé selon la revendication 1, caractérisé en ce que l'on prépare l'acide 1-(benzothiazol-2-ylamino)méthane-1,1-diphosphonique ou l'un de ses sels.

12. Procédé selon la revendication 1, caractérisé en ce que l'on prépare l'acide,1-(benzoxazol-2-ylamino)méthane-1,1-diphosphonique ou l'un de ses sels.

13. Procédé selon la revendication 1, caractérisé en ce que l'on prépare l'acide 1-(4-méthylthiazol-2-ylamino)méthane-1,1-diphosphonique ou l'un de ses sels.

14. Procédé selon la revendication 1, caractérisé en ce que l'on prépare l'acide 1-(5-méthylthiazol-2-ylamino)méthane-1,1-diphosphonique ou l'un de ses sels.

15. Procédé pour l'obtention de préparations pharmaceutiques, caractérisé en ce que l'on mélange un composé de formule

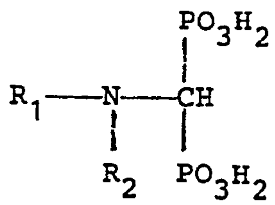

(I)

où $R_1$ représente les restes imidazolyle, benzimidazolyle, 2H-1, 2, 3- ou 4H-1, 2, 4-triazolyle, tétrazolyle, oxazolyle, benzoxazolyle, oxadiazolyle, thiazolyle, benzothiazolyle ou thiadiazolyle non substitués en C ou mono- ou disubstitués en C par un alkyle inférieur, un alcoxy inférieur, un phényle non substitué ou mono-ou disubstitué par un alkyle inférieur,par un alcoxy inférieur et/ou par un halogène, un hydroxy, un dialkyle inférieur amino, un alkyle inférieur thio et/ou un halogène et/ou monosubstitués en N sur un atome de N substituable éventuellement présent par un phénylalkyle inférieur non substitué ou de préférence mono-ou disubstitué par un alkyle inférieur, un alcoxy inférieur et/ou un halogène et $R_2$ représente l'hydrogène ou un alkyle inférieur ou où $R_1$ représente un reste pyrazol-3-yle ou isoxazol-3-yle non substitué ou mono-ou disubstitué par un alkyle inférieur et/ou par un halogène et $R_2$ représente l'hydrogène, où par restes inférieurs, on entend des restes présentant jusqu'à 7 atomes de carbone, ou l'un de ses sels pharmaceutiquement utilisables avec des adjuvants pharmaceutiques usuels.

16. Procédé pour l'obtention de préparations pharmaceutiques, caractérisé en ce que l'on mélange un composé obtenu selon l'une des revendications 1-14 avec des adjuvants pharmaceutiques usuels.

17. Procédé selon la revendication 15 pour l'obtention de préparations pharmaceutiques contenant l'acide 1-(pyrazol-3-ylamino)méthane-1,1-diphosphonique, l'acide 1-(4-méthylpyrazol-3-ylamino)méthane-1,1-diphosphonique, l'acide 1-(5-méthylpyrazol-3-ylamino)méthane-1,1-diphosphonique, l'acide 1-(isoxazol-3-ylamino)méthane-1,1-diphosphonique, l'acide 1-(4-méthylisoxazol-3-ylamino)méthane-1,1-diphosphonique ou l'acide 1-(5-méthylisoxazol-3-ylamino)méthane-1,1-diphosphonique ou l'un de leurs sels pharmaceutiquement utilisables.